(19) Europäisches Patentamt
European Patent Office
Office européen des brevets

(11) **EP 3 191 005 B1**

(12) **EUROPEAN PATENT SPECIFICATION**

(45) Date of publication and mention
of the grant of the patent:
**13.03.2024 Bulletin 2024/11**

(21) Application number: **15771355.3**

(22) Date of filing: **11.09.2015**

(51) International Patent Classification (IPC):
*A61B 18/20* (2006.01)   *A61B 18/00* (2006.01)
*A61B 5/00* (2006.01)

(52) Cooperative Patent Classification (CPC):
**A61B 18/20; A61B 18/22;** A61B 2018/00541;
A61B 2018/00559; A61B 2018/00589;
A61B 2018/00601; A61B 2018/00607;
A61B 2018/00636; A61B 2018/00702;
A61B 2018/00982; A61B 2018/00994;
A61B 2018/207

(86) International application number:
**PCT/US2015/049666**

(87) International publication number:
**WO 2016/040791 (17.03.2016 Gazette 2016/11)**

(54) **SYSTEMS AND METHODS FOR IMAGING AND MANIPULATING TISSUE**

SYSTEME UND VERFAHREN ZUR BILDGEBUNG UND MANIPULATION VON GEWEBE

SYSTÈMES ET PROCÉDÉS D'IMAGERIE ET DE MANIPULATION DE TISSU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.09.2014 US 201462049955 P**

(43) Date of publication of application:
**19.07.2017 Bulletin 2017/29**

(73) Proprietor: **Research Development Foundation
Carson City, NV 89703 (US)**

(72) Inventors:
• **FELDMAN, Marc, D.
San Antonio, TX 78229 (US)**
• **MILNER, Thomas, E.
Austin, TX 78712 (US)**

(74) Representative: **Grünecker Patent- und
Rechtsanwälte
PartG mbB
Leopoldstraße 4
80802 München (DE)**

(56) References cited:
WO-A2-2007/134256   WO-A2-2007/134257
US-A- 5 451 221   US-A1- 2005 113 641
US-A1- 2006 153 254   US-A1- 2011 257 641
US-A1- 2012 123 205   US-A1- 2012 310 042

## Description

## BACKGROUND INFORMATION

Traditional surgical techniques and apparatus have utilized independent systems and components for imaging and manipulating tissue. For example, a first system may be used to image the area in which the surgical technique is to be performed, while separate systems may be used to coagulate and cut the tissue.

The use of multiple independent systems can reduce the accuracy of the procedure, as the surgeon is not able to manipulate the tissue in spatial and/or temporal registration with preferred tissue visualization. In addition, the use of separate systems for independent functions performed during surgical procedures can result in increased surgical times as each system must be introduced and withdrawn from the surgical site, increasing patient morbidity and mortality. Other issues arising from the use of multiple surgical systems include limited access to surgical sites for multiple systems and increased costs.

Traditional systems and techniques may also use mechanical components for tissue manipulation that can lead to trauma to surrounding tissues and longer recovery times for patients. In addition, the inability to concurrently image and cut has resulted in the inability to perform certain operations, or to perform operations inadequately requiring repeat procedures in the future.

Many neurologic cancer surgeries require specialized tools that enhance imaging for precise cutting and removal of tumor cells and tissues without damage to adjacent benign structures. For example, brain tumors are accessible via the ventricles but inoperable because the surgeon cannot concurrently image the boundary between tumor and healthy tissue and establish a safe margin. Although prestaging imaging and surgical operating procedures have improved considerably over the last two decades, rate of successful outcomes (i.e., cancer-free patients) have not meaningfully improved. A leading hypothesis for the existing high recurrence rate and corresponding poor outcomes is that cancer cells are being left within the patient due to improper selection of tumor margins. Current methods utilized for tumor margin selection using traditional biopsy during surgery are limited and time-consuming. Current practice requires the surgeon to physically label tissue regions for biopsy, perform the biopsy, manually transfer tissue to a pathology laboratory, creation of frozen sections and sectioning, staining the frozen section, diagnostic observation of frozen tissue sections under a microscope by a cancer pathologist and finally oral communication of the diagnostic results back to the surgeon - a process that requires about thirty minutes and is disruptive to the surgeon's preferred workflow.

Accordingly, systems and methods are desired that overcome these and other limitations associated with existing systems and methods.

US 5,451,221 A discloses an endoscopic light delivery system for delivering light to tissue that includes a laser source for generating light. Fiber optics are optically coupled to the laser source for conveying the light generated by the laser source. A focusing surface is formed at an end of the fiber optics. The focusing surface of the fiber optics is shaped for redirecting light conveyed by the fiber optics in a direction lateral to the fiber optics for cutting tissue. A single optical fiber or a bundle of optical fiber can be used to transmit plural wavelengths of light for aiming cutting and coagulating.

US 2011/257641 A1 discloses an optical fibre arrangement for coagulating and ablating tissue targeted by an OCT system.

## SUMMARY

The present invention relates to a system as defined in independent claim 1 and a method of manipulating tissue ex vivo as defined in claim 13. Preferred embodiments are defined in the dependent claims.

The present disclosure includes systems and methods capable of imaging and manipulating tissue using light, including for example, coagulating and breaking the molecular bonds (*e.g.* cutting) tissue. Particular embodiments may be configured for use in neurosurgery, ear, nose and throat (ENT) procedures, obstetrics, gynecology, gastroenterology, lung procedures, peripheral nerve surgery, or other applications.

Exemplary embodiments utilize advantages of light energy to image and manipulate tissue, including simultaneously imaging and manipulation in some embodiments. For example, light has the capacity to transmit large amounts of encoded information (*e.g.* 300 THz) at sub-cellular spatial scales (1 $\mu$m). In addition, light can penetrate epithelial tissue layers without an incision, since light can modify a desired target by photon absorption or photon momentum transfer (scattering). Particular embodiments utilize optical coherence tomography (OCT) and multiphoton luminescence (MPL) systems and components to image the tissue, and lasers to provide light for manipulating tissue, including coagulating and breaking the molecular bonds of tissue.

Exemplary aspects of the present disclosure integrate three novel laser technologies for imaging, coagulation (*e.g.* blood flow interruption) and tissue removal (*e.g.* tissue "cutting") and inspire a new surgical paradigm. Capability for image-guided high speed tissue removal with a cutting precision of a few cell layers is unprecedented in the surgical arts. Exemplary aspects will allow access to remove previously inoperable tumors and other pathologic tissues in small confined spaces. Accordingly, surrounding nerves, specialized muscles and important glands can be spared, thereby substantially improving a patient's prognosis following surgery.

Exemplary embodiments may be utilized for surgical removal of many complex lesions in close proximity with specialized nerves, muscles, glands, and other normal organs and supporting structures. For example, endometriosis is frequently associated with the ovary, bowel and bladder, and other abdominal structures, and removal using conventional surgical procedures poses a risk of diminishing the longevity of fertility to the female patient due to a loss of ovum or eggs, or contamination of the abdomen with feces or urine. Exemplary embodiments may rapidly safely, and more precisely remove ovarian lesions sparing normal ovarian follicles, and remove endometrial tissue without the risk of bacterial contamination of the sterile abdominal cavity.

The invention includes a system as defined in claim 1, comprising inter alia: a first light source configured to provide a signal for use in imaging tissue when the first light source is incident upon tissue; a second light source configured to coagulate tissue (e.g. surrounding blood vessels); and a third light source configured to break molecular bonds of tissue coagulated by the second light source when the third light source is incident upon tissue. In particular embodiments, the first light source, the second light source and the third light source emit light through a single fiber (or larger structures such as an endoscope or laproscope) at the same instance. In some embodiments, the first light source, the second light source and the third light source emit light through a single fiber (or larger structures such as an endoscope or laproscope) at different times. In some embodiments the single fiber may be a component of an endoscope or laproscope. The signal obtained from the first light source may be used to orient or position the light source(s) used to perform the blood flow interruption and tissue removal functions. In some examples, the blood flow interruption (e.g. coagulation) and tissue removal functions may be performed by a separate second and third light source, while in other examples, the blood flow interruption and tissue removal functions may be performed by the same light source (e.g. the second light source).

The first light source comprises an OCT light source. In other embodiments, the first light source may comprise a short pulsed light source suitable for MPL, and in still other embodiments, the first light source may be utilized for both OCT and MPL. In certain embodiments, the third light source is a diode laser seeded fiber amplified sourcethat is configured to emit energy in a range of wavelengths from 1800 nm to 2200 nm. In some embodiments, the diode laser seeded fiber amplified source can be configured to emit energy having a pulse profile, a pulse energy, and a pulse repetition rate. In particular embodiments, at least one of the pulse profile, pulse energy and pulse repetition rate can be controlled to adjust a tissue removal rate.

In certain embodiments, the third light source is a tunable semiconductor laser seeded fiber amplified source, that is configured to emit energy in a range of wavelengths from 1800 nm to 2200 nm. In some embodiments, the tunable semiconductor laser seeded fiber amplified source can be configured to emit energy having a pulse profile, a pulse energy, and a pulse repetition rate. In specific embodiments, at least one of the pulse profile, pulse energy and pulse repetition rate can be controlled to adjust a tissue removal rate.

In certain embodiments, the third light source can be configured to break molecular bonds of tissue coagulated by the second light source when the third light source is incident upon tissue. In particular embodiments, the third light source can be configured to alter the quaternary structure of proteins of tissue when the third light source is incident upon tissue.

The signal(s) obtained from the first light source is (are) input into a computer processor. The computer processor provides output data used to control an orientation or position of the second light source and the third light source. In some embodiments, the computer processor provides output data used to control pulse profile, pulse energy and pulse repetition rate of the third light source. In certain examples, the second light source is a laser that emits energy in a range of wavelengths that are absorbed by blood. In specific examples, the blood comprises a mixture of oxy-hemoglobin, deoxy-hemoglobin and water. In some examples, the blood contains hemoglobin that comprises pure oxy-hemoglobin. In certain examples, the blood contains hemoglobin that comprises pure deoxy-hemoglobin. In particular embodiments, the second light source is a ytterbium fiber laser, a yttrium aluminum garnet (YAG) laser, a frequency-doubled ytterbium fiber laser, a frequency-doubled YAG laser, a dye laser, or a Tm fiber laser.

In certain embodiments, the second light source can be a frequency-doubled ytterbium fiber laser. In particular embodiments, the third light source can be a Tm doped fiber master oscillator power amplifier (MOPA). In some the Tm doped MOPA seed laser is can be a semiconductor diode laser. In specific embodiments, the seed laser can be a tunable laser.

The second light source is configured to emit energy in a range of wavelengths from 350 nm to 2200 nm. In certain embodiments, the second light source is configured to emit energy in a range of wavelengths including 532 nm, 585 nm, 1064 nm and/or 1940nm

In some embodiments, the optical coherence tomography light source is configured as a swept source optical coherence tomography light source. In specific embodiments, the optical coherence tomography light source is configured as a broadband optical coherence tomography light source. In some embodiments, the first light source comprises a multiphoton luminescence light source, and in particular embodiments the first light source comprises an optical coherence tomography light source and a multiphoton luminescence light source. In certain embodiments, the second light source is configured to emit energy at an amplitude and frequency sufficient to modify at least quaternary structure of tissue proteins without substantially breaking the molecular bonds of the tissue. In particular embodiments, the third light source is a laser configured to emit energy at an amplitude and frequency sufficient to break molecular bonds of tissue.

The present disclosure includes a system comprising: an imaging light source configured to provide data for use in imaging tissue when the first light source is incident upon tissue; a coagulating light source configured to emit coagulating light to coagulate tissue when the coagulating light is incident upon tissue; and a bond-breaking light source configured to emit bond-breaking light to break molecular bonds of tissue coagulated by the second light source when the bond-breaking light source is incident upon tissue. In some examples, the imaging light source comprises an optical coherence or a multiphoton luminescence light source, and in particular examples the imaging light source comprises an optical coherence tomography light source and a multiphoton luminescence light source.

In certain examples, the coagulating light and the bond-breaking light originate from a common light source. In particular examples, the common light source is a diode laser seeded fiber amplified source. In some examples, the diode laser seeded amplified source can be configured to emit energy in a range of wavelengths from 1800 nm to 2200 nm. In specific examples, the diode seeded fiber amplified source can be configured to emit energy having a pulse profile, a pulse energy, and a pulse repetition rate. In certain examples, at least one of the pulse profile, pulse energy and pulse repetition rate can be controlled to adjust tissue coagulation. In certain examples, at least one of the pulse profile, pulse energy and pulse repetition rate can be controlled to adjust a tissue removal rate.

In specific examples, the common light source can be a tunable semiconductor laser seeded fiber amplified source. In particular examples, the tunable semiconductor laser seeded fiber amplified source can be configured to emit energy in a range of wavelengths from 1800 nm to 2200 nm. In some examples, the tunable semiconductor laser seeded fiber amplified source can be configured to emit energy having a pulse profile, a pulse energy, and a pulse repetition rate. In specific examples, at least one of the pulse profile, pulse energy and pulse repetition rate can be controlled to adjust a tissue coagulation rate. In specific examples, at least one of the pulse profile, pulse energy and pulse repetition rate can be controlled to adjust a tissue removal rate. In certain examples, the bond-breaking light source can be configured to break molecular bonds of tissue coagulated by the coagulating light source when the bond-breaking light source is incident upon tissue.

In particular examples, the coagulating light source and the bond-breaking light source originate from a common light source at the same instance during use. In some examples, the coagulating light source and the bond-breaking light source originate from a common light source at different times during use. In specific examples, the coagulating light source and the bond-breaking light source originate from separate light sources.

The present disclosure further relates to a method of manipulating tissue, in which the method comprises: obtaining initial image signals of a first portion of tissue, wherein a first light source is incident on the first portion of tissue; positioning a second light source on a second portion tissue, wherein the second light source coagulates the second portion of tissue; and breaking molecular bonds of a third portion of tissue via light energy. In some aspects, the first light source comprises an optical coherence or a multiphoton luminescence light source, and in particular embodiments the first light source comprises an optical coherence tomography light source and a multiphoton luminescence light source.

In certain aspects, the second portion of tissue comprises the first portion of tissue and the third portion of tissue. Some aspects further comprise obtaining additional image data of the first portion of tissue after positioning the second light source on the second portion of tissue and before breaking the molecular bonds of the first portion of tissue. In specific aspects, the light energy used to break the molecular bonds of the third portion of tissue is emitted from a third light source. In certain aspects, the first light source, the second light source and the third light source emit light through a single fiber or larger structures such as an endoscope or laproscope. In specific aspects, the single fiber can be a component of an endoscope or laproscope. In particular aspects, the light energy used to break the molecular bonds of the third portion of tissue is emitted from the second light source.

Further examples include a method of modifying tissue, in which the method comprises: directing light energy to a first portion of tissue and recording image signals of the first portion of tissue; directing light energy to a second portion of tissue and coagulating the second portion of tissue; and directing light energy to a third portion of tissue and breaking molecular bonds of the third portion of tissue. In some examples, directing light energy to a first portion of tissue comprises directing light energy from an optical coherence tomography light source or a multiphoton luminescence light source. In particular examples, directing light energy to a first portion of tissue comprises directing light energy from an optical coherence tomography light source and a multiphoton luminescence light source. In certain examples, the second portion of tissue comprises the first portion of tissue and the third portion of tissue.

In particular examples, the light energy directed to the first portion of tissue is emitted from a first light source and the light energy directed to the second and third portions of tissue originate from a common light source. In some examples, the light energy directed to the first portion of tissue is emitted from a first light source; the light energy directed to the second portion of tissue is emitted from a second light source; and the light energy directed to the third portion of tissue is emitted from a third light source.

Other examples include a method of modifying tissue, in which the method comprises: positioning a first light source such that the first light source is incident on a first portion of tissue; obtaining initial image signals of the first portion of tissue; positioning a second light source such that the second light source is incident on a second portion of tissue, wherein the second light source coagulates the second portion of tissue; and breaking molecular bonds of a third portion of tissue. In some examples, the first light source comprises an optical coherence or a multiphoton luminescence light source, and in particular examples the first light source comprises an optical coherence tomography light source and a multiphoton luminescence light source. In some examples, imaging the first portion of tissue comprises directing light energy from an optical coherence tomography light source or a multiphoton luminescence light source to the first portion of tissue.

Certain examples include a method of modifying tissue, in which the method comprises: imaging a first portion of tissue; coagulating a second portion of tissue with light energy; and breaking molecular bonds of a third portion of tissue with light energy. In some examples, imaging the first portion of tissue comprises directing light energy from an optical coherence tomography light source or a multiphoton luminescence light source to the first portion of tissue. In certain examples, imaging the first portion of tissue comprises directing light energy from an optical coherence tomography light source and a multiphoton luminescence light source to the first portion of tissue. In particular examples, the first portion of tissue, the second portion of tissue, and the third portion of tissue contain common tissue. In certain examples, the first portion of tissue includes the second portion of tissue and the third portion of tissue. In particular examples, the light energy used for coagulating the second portion of tissue and the light energy used for breaking molecular bonds originate from a common light source. In some examples, the light energy used for coagulating the second portion of tissue and the light energy used for breaking molecular bonds originate from separate light sources.

Certain examples include a system comprising: a first light source configured to provide data for use in imaging tissue when the first light source is incident upon tissue; and a second light source configured to coagulate tissue and configured to break molecular bonds of when the second light source is incident upon tissue. In some examples, the first light source comprises an optical coherence or a multiphoton luminescence light source, and in particular examples the first light source comprises an optical coherence tomography light source and a multiphoton luminescence light source. In particular examples, the first light source and the second light source are configured emit light through a single fiber at the same instance. In some examples, the single fiber can be a component of an endoscope or laproscope. In specific examples, the first light source and the second light source are configured to emit light through a single fiber at different times. In particular examples, the single fiber can be a component of an endoscope or laproscope. In some examples, the data obtained from the first light source can be used to orient or position the second light source and the third light source. In specific examples, the data obtained from the first light source can be an input into a computer processor.

In certain examples, the computer processor can provide output data used to control an orientation or position of the second light source. In particular examples, the second light source is a laser that emits energy in a range of wavelengths that are absorbed by blood. In specific examples, the blood comprises a mixture of oxy-hemoglobin, deoxy-hemoglobin and water. In some examples, the blood comprises pure oxy-hemoglobin. In particular examples, the blood comprises pure deoxy-hemoglobin. In certain examples, the second light source can be a Tm doped fiber MOPA. In particular examples, the second light source can be a tunable Tm doped fiber MOPA. In particular examples, the second light source can be configured to emit energy in a range of wavelengths including 1940 nm. In certain examples, the second light source can be configured to emit energy in a range of wavelengths from 450 nm to 2200 nm. In particular examples, the first light source can be configured as a swept source optical coherence tomography light source. In some examples, the first light source can be configured as a broadband optical coherence tomography light source such as a superluminescent diode. In certain examples, the first light source may comprise a MPL light source, and particular examples, the first light source may comprise both OCT and MPL light sources. In specific examples, the second light source can be configured to emit energy at an amplitude and frequency sufficient to modify at least quaternary structure of tissue proteins and sufficient to break the molecular bonds of the tissue.

Certain examples include a tissue processing system comprising: a vacuum-assisted processing chamber configured to receive a tissue sample; a first micro-position actuator configured to position the tissue sample within the vacuum-assisted processing chamber; a second micro-position actuator configured to position the tissue sample within the vacuum-assisted processing chamber; and a laser configured to remove a portion of the tissue sample within the vacuum-assisted processing chamber. In particular examples, the laser is a femtosecond pulsed laser. In some examples, the first micro-position actuator and the second micro-position actuators are orthogonal to each other. In specific examples, the tissue-processing chamber comprises phosphate buffered saline, and in come examples the tissue-processing chamber comprises an optically transparent window. In certain examples, the tissue-processing chamber is disposable.

In particular examples, the tissue-processing chamber comprises a first self-sealing septum to form a water-tight seal for engagement of the first micro-position actuator; and the tissue-processing chamber comprises a second self-sealing septum to form a water-tight seal for engagement of the second micro-position actuator. In some examples, the first micro-position actuator comprises a needle configured to pierce the first self-sealing septum, and the second micro-position actuator comprises a needle configured to pierce the second self-sealing septum.

In specific examples, the first micro-position actuator is coupled to a first stepper motor, and the second micro-position actuator is coupled to a second stepper motor. In certain examples, the first and second stepper motors can be operated to rotate the tissue sample within the vacuum-assisted processing chamber.

Particular examples include a method for analyzing tissue, where the method comprises: harvesting a tissue sample from a specimen via a vacuum-assisted instrument; transporting the tissue sample through a positive-pressure fluidic channel to a tissue processing chamber; cutting a section from the tissue sample; performing a multiphoton luminescence - optical coherence tomography (MPL-OCT) optical assay on the section; and processing data from the MPL-OCT optical assay. In some examples, the time elapsed between harvesting the tissue sample and processing data from the MPL-OCT optical assay is less than five minutes. In specific examples, a field programmable gate array (FPGA) processes from the MPL-OCT optical assay. In certain examples, processing data from the MPL-OCT optical assay comprises establishing a tumor margin within the section of the tissue sample. In some examples, the tumor margin comprises overlaying a first image of the section and a second image of the section. In specific examples, the section is approximately 500 $\mu$m thick. In particular examples, the MPL-OCT optical assay is performed on a first side of the section and a second side of the section. In some examples, the tissue sample is between 1.0 and 4.0 cubic millimeters in volume. In certain examples, the vacuum-assisted instrument is integrated into a scalpel. In particular examples, the vacuum-assisted instrument is integrated into a cautery.

In the following, the term "coupled" is defined as connected, although not necessarily directly, and not necessarily mechanically.

The use of the word "a" or "an" when used in conjunction with the term "comprising" in the claims and/or the specification may mean "one," but it is also consistent with the meaning of "one or more" or "at least one." The term "about" means, in general, the stated value plus or minus 5%. The use of the term "or" in the claims is used to mean "and/or" unless explicitly indicated to refer to alternatives only or the alternative are mutually exclusive, although the disclosure supports a definition that refers to only alternatives and "and/or."

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a method or device that "comprises," "has," "includes" or "contains" one or more steps or elements, possesses those one or more steps or elements, but is not limited to possessing only those one or more elements. Likewise, a step of a method or an element of a device that "comprises," "has," "includes" or "contains" one or more features, possesses those one or more features, but is not limited to possessing only those one or more features. Furthermore, a device or structure that is configured in a certain way is configured in at least that way, but may also be configured in ways that are not listed.

Other objects, features and advantages of the present invention will become apparent from the following detailed description. It should be understood, however, that the detailed description and the specific examples, while indicating specific embodiments of the invention, are given by way of illustration only.

## BRIEF DESCRIPTION OF THE DRAWINGS

The patent or application file contains at least one drawing executed in color. Copies of this patent or patent application publication with color drawing(s) will be provided by the Office upon request and payment of the necessary fee.

The following drawings form part of the present specification and are included to further demonstrate certain aspects of the present disclosure. The invention may be better understood by reference to one of these drawings in combination with the detailed description of specific embodiments presented herein.

FIG. 1 shows a schematic of an apparatus according to an exemplary embodiment.
FIG. 2 shows a schematic of an apparatus according to an exemplary embodiment.
FIG. 3 shows a schematic of an apparatus according to an exemplary embodiment.
FIG. 4 shows a schematic of an apparatus according to an exemplary embodiment.
FIGS. 5A-5C show a schematic of a light source according to an exemplary embodiment of a light source
FIG. 6 shows a schematic of components of the light source of FIG. 5A.
FIG. 7 shows a schematic of components of the light source of FIG. 5A.
FIG. 8 shows a schematic of components of the light source of FIG. 5A.
FIG. 9 shows a schematic of an apparatus according to an exemplary embodiment with an X-Y scanning galvanometer.
FIG. 10 shows a particular embodiment of the delivery system according to an exemplary embodiment.
FIG. 11 shows images taken according to exemplary embodiments.

FIG. 12 shows a schematic of an apparatus comprising an endoscopic or laproscopic micro-applicator according to an exemplary embodiment.

FIG. 13 shows a schematic of a laser-based optical biopsy system.

FIG. 14 shows a schematic of a tissue processing system.

## DETAILED DESCRIPTION OF ILLUSTRATIVE EMBODIMENTS AND EXAMPLES

Exemplary embodiments of the present disclosure include systems and methods, as defined in the claims, that utilize light to image tissue, coagulate tissue (e.g. blood vessels), and break molecular bonds of (e.g. cut) the tissue. The light used to perform each of these tasks may be produced by independent and separate components, including for example, lasers. In other embodiments, a single component (e.g. laser) may be configured to produce light energy that is used to perform multiple tasks, including coagulating the tissue and breaking molecular bonds of the tissue. It is understood that the embodiments described herein are merely exemplary, and that other embodiments are included within the scope of the invention that is defined by the claims.

As used herein, the term "coagulate" (and related terms, such as "coagulating", "coagulation", etc.) is used to refer to a process of interrupting blood flow (e.g. by damaging blood vessels so that when cut, blood does not flow outside the tissue) and rearranging and/or restructuring molecular bonds in tissue without completely breaking a majority of the molecular bonds. Also as used herein, the term "cut" (and related terms such as "cutting", etc.) is used to refer to a process of breaking the molecular bonds in tissue.

As used herein, the term "light source" is understood to include any source of electromagnetic radiation, including for example, a laser. It is also understood that a "first light source" and a "second light source" may originate from a single laser. For example, a laser configured for operating under a first set of parameters (e.g. wavelength, amplitude, continuous wave or continuous pulse mode) may be considered a "first light source", while the same laser configured for operating under a second set of parameters may be considered a "second light source."

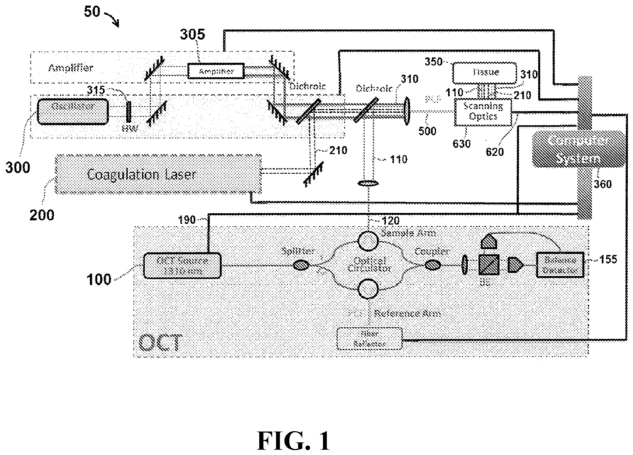

Referring now to FIG. 1, one exemplary embodiment of a system 50 comprises a first light source 100, a second light source 200 and a third light source 300. In the embodiment shown, first light source 100 (with associated optical components described more fully below) are configured as an optical coherence tomography (OCT) system configured to emit light 110 and provide a signal for use in imaging tissue 350 when the first light source 100 is incident upon tissue 350.

In addition, second light 200 source can be configured to emit light 210 and coagulate tissue 350 when second light 200 source is incident upon tissue 350. Also shown in system 50, third light source 300 can be configured to emit light 310 and break molecular bonds of tissue 350 coagulated by second light source 200 when third light source 300 is incident upon tissue 350.

In certain embodiments, system 50 can be configured such that first light source 100, second light source 200 and third light source 300 emit light through a single fiber 500 (e.g. a photonic crystal fiber) at the same instance (e.g. simultaneously) or at different times during use. A signal 190 obtained from first light source 100 can be used to orient or position second light source 200 and third light source 300 (and/or to orient or position light emitted from second light source 200 and third light source 300). The signal 190 obtained from first light source 100 is an input into a computer processor 360.

The computer processor 360 provides output data 620 to scanning optics control module 630 used to control an orientation or position of second light source 200 and third light source 300 (or an orientation or position of light emitted from such light sources). In certain embodiments, signals from first light source 100 may also be used to control pulse duration, pulse energy or pulse repetition rate of either second light source 200 or third light source 300.

The first light source 100 can produce near-infrared light, and the use of relatively long wavelength light allows deeper penetration into the scattering medium such as tissue 350. The first light source 100 can be configured to provide light at a wavelength of approximately 1310 nm.

As light in sample path 120 is directed at tissue 350, a small portion of this light that reflects from sub-surface features of tissue 350 is collected. During operation, a significant portion of light in sample path 120 is not reflected but, rather, backscatters from the sample. Although backscattered light contributes background that obscures an image in conventional imaging, this light can be used beneficially in OCT systems via interferometry. For example, balanced detector 155 can be used to record the optical path length of received photons, allowing rejection of most photons that multiply scatter in the tissue before detection. This can allow recording three-dimensional images of thick samples to be constructed by rejecting background signal while collecting light directly reflected from regions of interest in tissue 350. In an example, OCT imaging is generally limited to one to two millimeters below the surface in scattering biological tissue 350. At greater depths, the proportion of light that returns to the collection aperture with a single backscattering event is reduced making detection more challenging.

In examples in which first light source 100 comprises a MPL light source for, as light in sample path 120 is directed at tissue 350, a small portion of this light that is an endogenous fluorescence emission from sub-surface features of tissue 350 is collected. By collecting this endogenous multi-photon fluorescent emission signal, specific tissue components can be detected.

In certain examples, second light source 200 is a laser that emits energy in a range of wavelengths, including those that are absorbed by blood. In certain instances, the blood may comprise pure deoxy-hemoglobin and water, pure oxy-hemoglobin and water, or a mixture of oxy-hemoglobin, deoxy-hemoglobin and water.

In some examples, third light source 300 may emit light in a range between approximately 600 nm and 2200 nm. Light source 300 may also be used in conjunction with an amplifier 305, which may be a dispersion compensated chirped pulse amplifier. In particular examples, light emitted from light source 300 may pass through a half wave plate 315 prior to amplifier 305.

During operation, system 50 can therefore direct light configured to image, coagulate, and break molecular bonds (*e.g.* cut) of tissue 350. Specifically, light 110 from light source 100, light 210 from light source 200, and light 310 from light source 300 can allow a user to respectively image, coagulate, and break molecular bonds of tissue 350 in a single system without changing systems.

This can provide for numerous advantages over existing systems. For example, a user may be able to more accurately image, coagulate and cut tissue when the user does not have to utilize separate systems to perform separate functions during a surgical procedure. The accuracy may be improved, for example, because the user is not required to remove an imaging component from the site of interest and then introduce a second component to coagulate the tissue, followed by a cutting component. Successive introduction and reintroduction of components to image, coagulate and cut tissue can destroy spatial and temporal registration between these processes and compromise the surgical outcome.

In addition, the use of a single system to perform multiple functions can reduce the amount of time required to perform the surgical procedure, which can result in increased safety (for example, by reducing the amount of time the patient is required to be under anesthesia), and improved morbidity and mortality. Reducing the amount of time required for the surgical procedure can also reduce the associated costs. Finally, simultaneous imaging, coagulation to keep the imaging field clear of obstructing blood, and cutting can allow operations on previously inoperable pathologies as well.

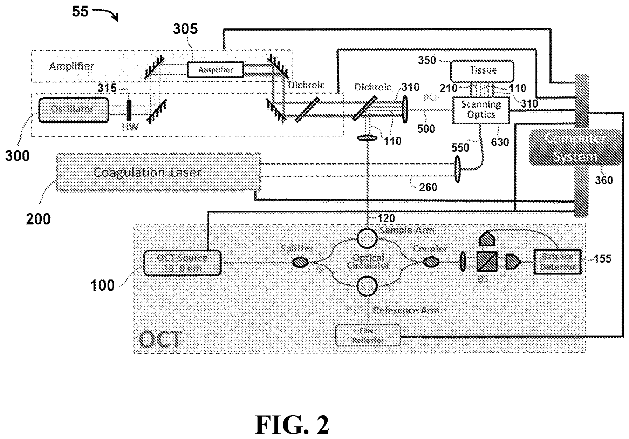

Referring now to FIG. 2, a system 55 is illustrated this is generally equivalent to the previously-described system 50 of FIG. 1. However, system 55 differs from system 50 in that second light source 200 directs light 260 through a fiber 550 that is independent of fiber 500. Fiber 550 (rather than fiber 500) can direct light 260 to scanning optics control module 630. Other aspects of FIG. 2 are generally equivalent to those shown in FIG. 1, and equivalent components are labeled according to the nomenclature used in FIG. 1.

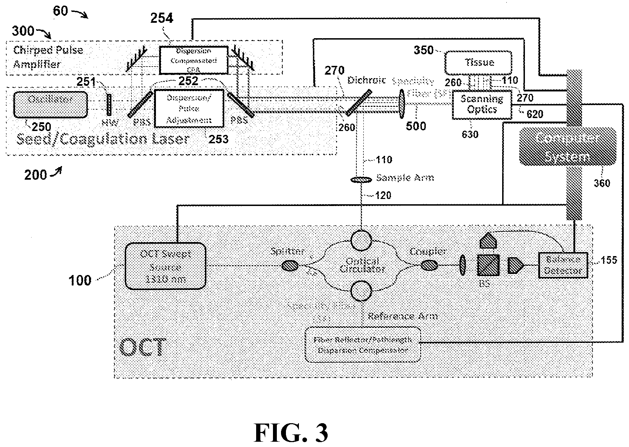

Referring now to FIG. 3, a system 60 is illustrated that is similar in many respects to the previously-described system 50 of FIG. 1. For example, like system 50, system 60 utilizes light to image, coagulate and break molecular bonds of tissue 350. However, system 60 does not comprise separate lasers for the light sources to provide light to coagulate and break molecular bonds of tissue 350. Instead, system 60 utilizes a single laser 250 (*e.g.* a seed laser) that can be controlled to function as light source 200 to provide light for the coagulating function and as light source 300 to provide light for the molecular bond breaking function. Laser 250 can be a frequency tunable laser, including for example a frequency tunable semiconductor laser comprising an intracavity tuning element. A specific example of such a laser is disclosed in U.S. Patent 7,415,049.

The use of a frequency tunable laser can provide advantages for tissue removal. For example, tuning the laser 250 can allow provide different ablation depths so that the tissue removal rate can be dynamically controlled. Tunable lasers configured for such use are available at a relatively low cost, and in a relatively small size. In certain examples, laser 250 can be controlled to provide separate light beams used to coagulate tissue and to break the molecular bonds of the tissue. In still other examples, the same laser source can emit light beams used to coagulate the tissue and also break the molecular bonds of the tissue (*e.g.* cut or ablate the tissue). In certain examples in which the same laser source is used to coagulate and break molecular bonds of the tissue, the parameters (*e.g.* pulse energy and pulse duration) of the light beams used to coagulate the tissue can be different than those used to break molecular bonds of the tissue.

In certain examples, laser 250 can be configured to provide light energy in a range of 600-2200 nm. Light emitted from laser 250 can be manipulated (*e.g.* via half wave plate 251, polarized beam splitters 252 and dispersion pulse adjustment 253) to provide light 260 suitable for coagulating tissue 350. In particular examples, light 260 is provided at an amplitude and frequency sufficient to modify at least quaternary structure of tissue proteins without substantially breaking the molecular bonds of tissue 350.

In addition, light emitted from laser 250 can also be amplified by dispersion compensated chirped pulse amplification 254 to provide light suitable 270 for breaking molecular bonds of tissue 350. Similar to the previously-described, light 110, 260 and 270 can be directed through single fiber 500. In addition, computer processor 360 can provide output data 620 to scanning optics control module 630 used to control an orientation or position of laser 250 and (and/or an orientation or position of light 260 and 270 emitted from such laser 250).

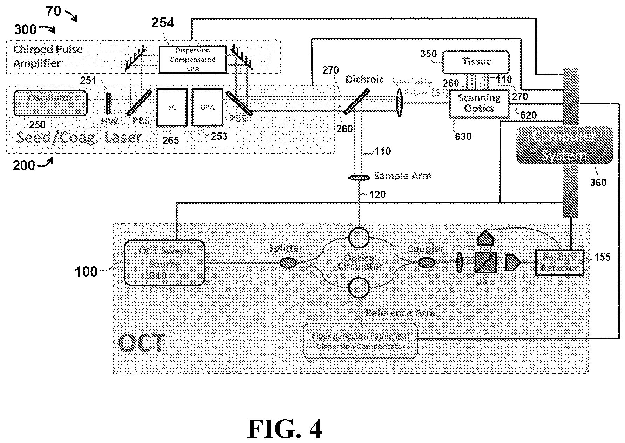

Referring now to FIG. 4, a system 70 is illustrated that is similar to system 60 previously shown and described in FIG. 3. In this example, however, system 70 comprises laser 255 that operates with a frequency converter (*e.g.* frequency doubler) 265. Other aspects of FIG. 4 are generally equivalent to those in FIG. 3, and equivalent components are labeled according to the nomenclature used in FIG. 3.

Light source 200 (*e.g.* the light source configured to coagulate tissue) may comprise an ytterbium fiber laser, a frequency-doubled ytterbium laser, an yttrium aluminum garnet (YAG) laser, a frequency-doubled YAG laser, a Tm fiber laser or a dye laser. Light source 200 may be configured to emit energy in a range of wavelengths including from approximately 350 nm to 2200 nm, and in specific embodiments, 532 nm, 585 nm, 1064 or 1940 nm. In particular examples, the light source configured to coagulate tissue may be configured as a continuous wave laser (*e.g.*, either emitting light continuously at a near constant level, or continuously pulsing).

It is understood that first light source 100, second light source 200 and third light source 300 may direct light to different portions of tissue 350. For example, first light source 100 and second light source 200 may direct light to a portion of tissue 350 that covers a larger area than the portion of tissue in which light source 400 directs light.

One design consideration for examples of the present disclosure is that the energy level of the light being transmitted through a transmission conduit (*e.g.* an optical fiber) should be low enough that it does not damage the fiber (*e.g.* fiber 500).

For example, a typical optical fiber can have a damage threshold of approximately 1 GW/cm². If one assumes a pulse duration of 1 picosecond and a fiber core diameter of 35 $\mu$m, the maximum pulse energy that could pass through fiber without causing damage would be 38 nJ. Accordingly, it is possible that very short pulsed lasers (*e.g.* picosecond or femtoseond) may have difficulty to provide sufficient pulse energy delivery inside the body through a fiber required for a desired tissue removal rate. In these cases, short-pulsed light energy can be delivered through the air using a reticulated arm. Light delivery using reticulated arms is well known in the art and has been used for decades to deliver, for example, 10.6 um light emitted from a Carbon Dioxide laser.

Additional design specifications of the "cutting beam" (*e.g.* the light beam used to break molecular bonds) can include for example, pulse energy ($E_p$), average power ($P_{ave}$), pulse repetition frequency ($f_R$), pulse duration ($\tau_p$), micro-applicator delivery to internal body sites, co-propagation with imaging and coagulation beams, and rapid tumor removal without damage to adjacent structures. One primary cutting beam design specification is the tissue removal rate ($V_R$ in mm³/s). A sufficiently high tissue removal rate needs to be supported for surgeons to effectively use examples of the present disclosure with procedure times that are not excessive.

To provide for acceptable procedure times, an initial target design specification for the tissue removal rate is $V_R >\_$ 100 mm³/s. Such a rate would allow a 1cm³ tumor mass or pathologic tissue to be removed in less than 10 seconds. Although faster tumor or pathologic tissue removal rates may be possible, a value of $V_R \geq$ 100 mm³/s represents a reasonable compromise between opposing design constraints.

An additional consideration in the configuration of exemplary systems and methods is that the light source or light sources used for tissue manipulation should provide a sufficient tissue removal rate while also providing precise control of the tissue removal. Higher tissue removal rates can provide for reduced procedure times, which can lower the risk of complications during the procedure and improve morbidity and mortality. Precise control over the light source (and/or the light emitted from the light source) can allow a surgeon to remove tissue that is desired to be removed, without removing surrounding tissue. A formula for tissue removal is provided below:

$$ V_R = \frac{\pi D_C^2 \cdot f_r}{4\mu_a} \cdot \ln\left(\frac{\Phi_o}{\Phi_{th}}\right) \qquad (1) $$

Where $V_R$ is the tissue removal rate (mm³/s), Dc is the diameter of the light source used for tissue manipulation in mm, $f_r$ is the pulse repitition frequency of the light source in Hz, $\mu_a$ is the tissue absorption rate (*e.g.* 0.0463 mm⁻¹), $\Phi_o$ is the light source incident fluence (J/cm²) on the tissue to be removed, and $\Phi_{th}$ is the threshold fluence (*e.g.* 4.3 J/cm²) for tissue ablation.

Equation (1) shown above can be used to establish light beam design specifications. For example, assume a $V_R$ of 100 mm³/s and a Thulium (Tm) Master Oscillator Power Amplifier (MOPA) with a pulse energy of 6.4 mJ to provide an incidence fluence *3x* ($\Phi_o$ = 12.9 J/cm²) above threshold. Based on these parameters, the cutting beam diameter on the tumor or pathologic tissue is calculated to be $D_C$ = 220 $\mu$m. A Tm MOPA that provides 12.9 J/cm² can remove a tumor or pathologic tissue thickness of about 216 $\mu$m for each absorbed pulse.

For an example with a tissue removal rate ($V_R$) exceeding 100 mm³/s, a pulse repetition frequency of $f_R$ =15.4kHz is needed. The design specifications of the Tm MOPA can be established, including cutting beam including pulse energy ($E_p$), average power ($P_{ave}$), and pulse duration ($\tau_p$) that allow rapid tumor removal rates ($V_R \geq$ 100 mm³/s) without damage to adjacent structures, micro-applicator delivery to internal body sites, and co-propagation with imaging and blood flow interruption beams. Certain examples can be configured such that the cutting beam can cut (*e.g.* break molecular bonds of) tissue below the surface tissues without disturbing the surface tissue layer.

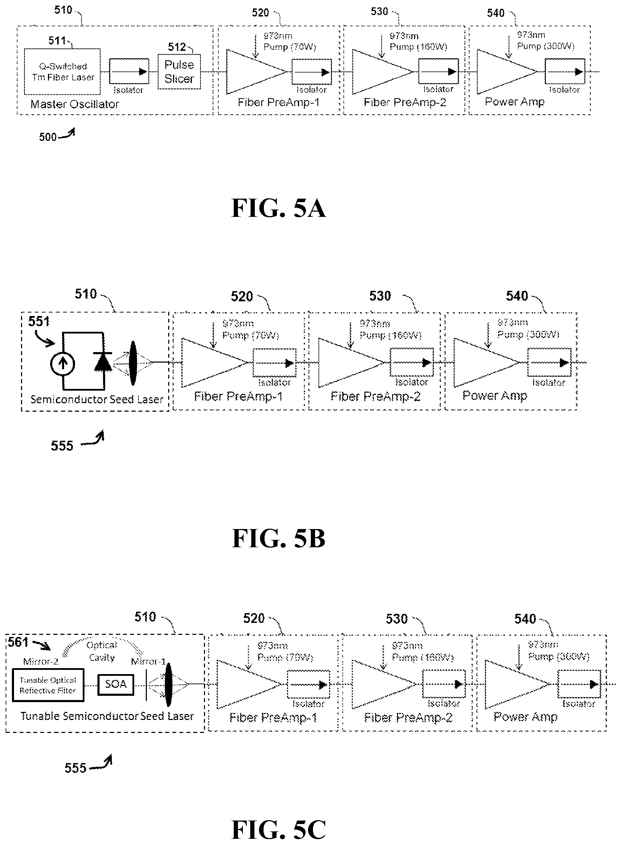

Referring now to FIG. 5A, an exemplary light source 555 is illustrated according to the principles described above. Light source 555 is configured to break molecular bonds of tissue when the light source is incident upon tissue (*e.g.* light source 555 is configured to provide light that can serve as a "cutting beam.") In the example shown in FIG. 5A, light source 555 is Thulium (Tm) Master Oscillator Power Amplifier (MOPA) laser comprising a master oscillator 510, which is configured to provide a seed pulse (at for example 1.95 $\mu$m). In addition this exemplary light source 555 comprises a first pre-amplifier 520 and a second pre-amplifier 530, as well a power amplifier 540.

In examples, master oscillator 510 may be configured in one of a number of different arrangements. For example, in certain examples, master oscillator 510 may comprise as a Q-switched Tm fiber laser 511, an optical isolator 529 and a pulse slicer 519 as shown in FIG. 5A. In certain examples, the pulse duration emitted from the Q-switched Tm fiber laser may be approximately 100 nanoseconds in duration. The pulse slicer can extract a sub-pulse of shorter duration from the 100 nanosecond pulse. For example, the pulse slicer may extract a 6 nanosecond pulse from the pulse emitted from the Q-switched Tm fiber laser 511. In the example shown in FIG. 5A, the 6 nanosecond pulse then enters first pre-amplifier 520 and second pre-amplifier 530 to increase power. In certain examples, the power can be increased to an average power of $P_{ave}$ = *100W* with a pulse energy of $E_p$ = 6.4 mJ and a pulse repetition frequency of $f_R$ = 15.4 kHz.

Referring now to FIG. 5B, in other examples, master oscillator 510 may be configured as a semiconductor diode laser 551 that can be gain switched with a pulsed current source to achieve the desired pulse duration. By gain switching semiconductor diode laser 551 (*e.g.*, InGaAs/InP or InSb) picosecond and nanosecond pulse durations may be achieved and the pulse repetition rate may be readily varied. Gain switched semiconductor diode lasers have been utilized to seed fiber amplifiers 520, 530 and provide average power levels of several hundred watts with peak powers up to 270kW and pulse durations in the picosecond to nanosecond range. Moreover, the use of a gain switched semiconductor diode laser allows for shaping the light pulse by adjusting the time variation of the injection current. The use of a semiconductor diode laser to provide the seed pulse can allow the pulse duration to be programmable since it is controlled by the injection current driven by the system electronics. For example, if a 3 nanosecond pulse with a given profile is desired, the semiconductor diode laser can be gain switched for a sufficiently short time period so that the amplified seed pulse is 3 nanoseconds. If a shorter pulse is desired, for example 200 picoseconds, the semiconductor diode laser can be gain switched for the appropriate time duration. Accordingly, semiconductor diode laser 551 can be precisely controlled to provide the desired pulse profile and duration pulse energy and pulse repetition rate. *See* Alexander M. Heidt, Zhihong Li, and David J. Richardson, "High Power Diode-Seeded Fiber Amplifiers at 2 $\mu$m-From Architectures to Applications" IEEE J. Select. Topics Quantum Electron., Vol. 20, No. 5, Sep./Oct. 2014. Selection of the pulse profile, pulse energy [*i.e.*, spot size ($D_C$)] and pulse repetition rate ($f_R$) allows control of the tissue removal rate ($V_R$).

Referring now to FIG. 5C, in other examples of light source 555, master oscillator 510 may be configured as a frequency tunable semiconductor diode laser 561 (*e.g.*, InGaAs/InP or InSb) that can be operated in either a gain switched or mode locked configuration to achieve a desired pulse duration ranging from femtoseconds, to picoseconds to longer times. A frequency tunable semiconductor diode laser incorporates a tunable filter in the laser cavity that constrains the laser to oscillate over a user-controlled narrow wavelength range (see for example U.S. Patent 7,415,049). The use of a frequency tunable laser can provide important advantages for tissue removal. For example, the absorption coefficient of tissue varies by a factor of about 20x over the 1800 - 2200 nm spectral range. Tuning the laser emission to a wavelength in the 1800 - 2200 nm spectral range allows different ablation depths in tissue and may be used to optimize coagulation and/or tissue removal rate by adjusting laser operating constraints including the emission wavelength, pulse repetition rate, and pulse energy. Selection of the pulse profile, pulse energy, spot size [*i.e.*, spot size ($D_C$)], pulse repetition rate ($f_R$) and laser emission wavelength [i.e., tissue absorption coefficient, $\mu_a(\lambda)$] allows control of the tissue removal rate ($V_R$).

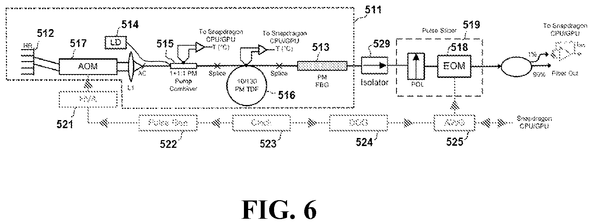

Referring now to FIG. 6, portions of light source 555 of FIG. 5A are shown in further detail to illustrate particular components of master oscillator 510 and laser 511. In this example, master oscillator 510 comprises Q-switched Tm fiber laser 511 (*e.g.* 1.95 $\mu$m) with the cavity formed between a high reflector (HR) 512 and an inline polarization-maintaining fiber Bragg grating (PM FBG) 513 shown in FIG. 6. The example shown also comprises an acousto-optic modulator (AOM) 517, a laser diode 514 (*e.g.*, a 35 W fiber coupled laser 973nm diode), a polarization maintaining pump combiner 515, and a polarization maintaining Tm doped fiber with 10 $\mu$m core and 130 $\mu$m cladding (PM TDF) 516. The example shown also comprises a pulse slicer 519 that includes an electro optic modulator (EOM) 518. The illustrated examples also comprise a high voltage amplifier (HVA) 521, a pulse generator 522, a clock 523, a digital delay generator (DDG) 524, and an arbitrary waveform generator (AWG) 525.

In the example shown, the AOM 517 is configured to function as the Q-switch which can dump the oscillator at a pulse repetition frequency $f_R$ =15.4kHz. In addition, optical isolator 529 can prevent pulse instabilities resulting from parasitic feedback into the cavity. The delay and duration of each pulse-slice can be fixed by EOM 518 driven AWG 525 as triggered by DDG 524. In certain examples, risk based design controls (*e.g.* according to FDA 21 CFR QSR 820.30) can be implemented by monitoring pulse diagnostics and the temperature of PC 515 and PM TDF 516.

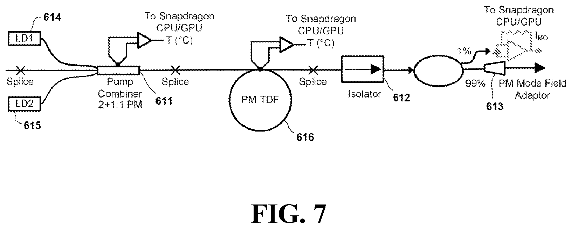

Referring now to FIG. 7, an exemplary architecture for the preamp-1 and -2 gain stages shown in FIG. 5 is provided. In this example, the preamp-1 comprises polarization maintaining Tm doped fiber (PM TDF) 616

with a 10 $\mu$m core and a 130 $\mu$m cladding pumped by a 35W fiber coupled laser (973 nm) diode LD1 614. Similarly, in this example, preamp-2 can comprise a polarization maintaining Tm doped fiber (PM TDF) with a 50 $\mu$m core and a 250 $\mu$m cladding pumped by an 80W fiber coupled laser (973 nm) diode LD2 615. The example shown also comprises a pump combiner 611, an isolator 612, and a polarization maintaining mode field adaptor 613.

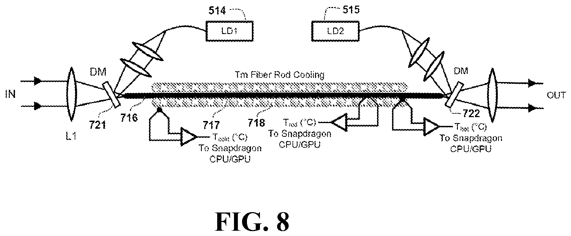

Referring now to FIG. 8, a schematic of power amp 540 (shown in FIG. 5) is provided. In this example, a Tm fiber rod 716 provides the final stage of pulse amplification giving, for example, $E_p$ = 6.4 mJ pulses at $f_R$ =15.4kHz corresponding to $P_{ave}$ = 100W of average power. In particular examples, Tm fiber rod 716 can be potted in a V-groove with thermally conductive epoxy 717 in a water-cooled copper plate 718. In the example shown, the Tm doped fiber-rod Power Amp gain stage is pumped by 200W (973nm) laser diode (LD1) 714 and 100W (973nm) laser diode (LD2) 715 reflected from dichroic mirrors (DM) 721, 722.

Design specifications of exemplary tissue removal light sources (*e.g.* "cutting" light sources) include pulse energy ($E_p$), average power ($P_{ave}$), pulse repetition frequency ($f_R$), pulse duration ($\tau_p$), and tissue removal rate ($V_R$). The pulse energy ($E_p$) can for example be determined by measuring the average power ($P_{ave}$) and the pulse repetition frequency ($f_R$). In addition, the pulse duration ($\tau_p$) of light exiting the Tm fiber rod power amplifier can be measured using a high-speed InGaAs photodiode and PicoScope 9211A oscilloscope.

The tumor or pathologic tissue removal rate ($V_R$ in mm³/s) can be measured using *ex vivo* tissue. For example, pathologic tissue specimens can be placed on a two-axis translation stage and raster scanned over a 5 $\times$ 5 mm² area in a 5 second time period while the tissue removal light source performs a cutting operation. After cutting, pathologic tissues can be placed under an OCT imaging system and tissue removal rate will be determined by measuring depth ($\Delta z$, *mm*) of ablation tissues using the relationship, $V_R$ = 20·$\Delta z$ (mm³/s). Proceeding in this manner, performance of the tissue removal light source can be verified. Similar studies can be performed in animal models of established pathologic tissues.

Validation of exemplary system parameters may first employ a bulk applicator before surgery is performed through an endoscopic or laproscopic micro-applicator. For example, design specifications of the bulk-applicator may be established before construction and verification of an endoscopic or laproscopic micro-applicator. In addition, design specifications of a user (surgeon) interface may be established and then validated. Finally, operation and performance of examples for pathologic tissue removal in an animal model of established pathologic tissues may be performed to validate the device.

Design specifications of the bulk applicator include diameters of imaging ($D_{OCT}$), blood flow interruption ($D_G$) and cutting ($D_C$) beams at the tissue surface. Cutting beam diameter ($D_C$ = 220 $\mu$m) may be established from the tissue removal rate ($V_R \geq$ 100 mm³/s), while $D_{OCT}$ may be determined by user required scan depth (*e.g.* 2 mm) and corresponding Rayleigh range of imaging light (1.31$\mu$m) giving $D_{OCT}$ = 25um. $D_G$ may be determined by available power (W) of the blood flow interruption laser (*e.g.*, 0.532 $\mu$m) and other design requirements to prevent bleeding.

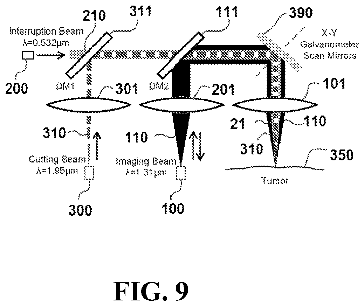

In examples the coagulation light source (*e.g.* the light source configured to interrupt blood flow) can be configured to treat vessels throughout the targeted cutting depth (*e.g.* the upper 200 $\mu$m of a tumor or other targeted tissue) corresponding to a single pass of the tissue removal light source (*e.g.* the cutting beam). Referring now to FIG. 9, the coagulation light source 200 (*e.g.* a 10W 0.532 $\mu$m laser) can provide 5W of optical power incident on tissue 350 to be removed (*e.g.* a tumor or pathologic tissue). A temperature increase in a large diameter (30 $\mu$m) vessel positioned 200 $\mu$m below the previously ablated tissue surface following a 25 $\mu$s exposure suggests that $D_G$ = 50 $\mu$m is a suitable design parameter to interrupt blood flow in tumor vessels.

To achieve the specified beam diameters on the surface of tissue 350 ($D_C$ = 220 $\mu$m, $D_{OCT}$ = 25 $\mu$m and $D_G$ =50 $\mu$m) in the bulk applicator, each beam should be collimated and incident on an X-Y scanning galvanometer 390 as shown in FIG. 9. Tissue removal beam 310 and imaging beam 110 reflect from dichroic mirrors 311 (DM1) and 111 (DM2), respectively, and are co-aligned with coagulation beam 210. From the user defined specified diameters on the surface of tissue 350, collimated beam diameters on the scan lens 301, 101, and 201 (*e.g. f* = 18 mm) are determined for tissue removal (0.2 mm), imaging (1.2 mm) and coagulation (0.25mm) beams respectively. Bulk applicator components including galvanometers, dichroic mirrors, and collimating lenses may be mounted in a stainless steel enclosure, *e.g.* fabricated by 3D rapid prototyping.

Operation of the bulk applicator may be verified by measuring diameters of cutting, imaging and interruption beams at the back focal plane of the scan lens for different field positions using a knife-edge technique. Scan speed of the bulk applicator will be verified by placing a frosted glass plate in the focal plane of the scan lens and recording time of flight of the coagulation laser beam spot with a high speed camera.

In some applications an applicator is desired such that the spot size of the imaging, coagulation and cutting beams incident on the tissue have a similar numerical aperture that is constrained by the beam delivery optics. For example, when the imaging, coagulation and cutting beams propagate through a common fiber the spot size of each beam on the tissue is constrained by the delivery optics. In this case, the OCT imaging beam diameter on the tissue is constrained by the imaging depth that is approximately given by twice the Rayleigh range of the beam. For example, for an imaging beam

wavelength of 1.31μm and a scan range of about 2mm, the imaging beam diameter on the tissue is about 20μm. For a cutting beam fluence of 8.6J/cm² a pulse energy of 35.8 uJ is required. By using a gain switched semiconductor diode laser as a seed source followed by staged Tm fiber amplifiers, a pulse repetition rate of about 1MHz may be achieved giving a volumetric tissue removal rate of more than 250 mm³/s. Such a configuration has important advantages over an endoscopic or laproscopic micro-applicator since the imaging, coagulation and cutting beams may be delivered through a common optical fiber so that a substantially smaller and more flexible applicator can be utilized.

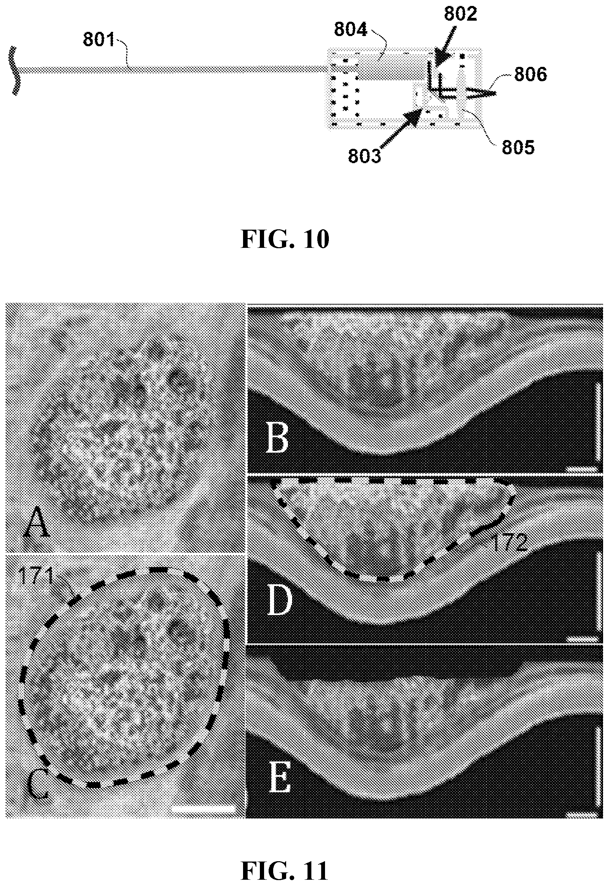

A particular example of the delivery system 800 is shown in FIG. 10. A fiber 801 (*e.g.*, photonic crystal fiber) propagates the imaging, coagulation and cutting beams that are collimated. A reflecting prism 802 directs light onto an X-Y MEMS scanning mirror 803 that is positioned in the back focal plane of the lens. The collimator 804 and scanning lens 805 form a 4f imaging system so that the fiber tip is conjugate to the back focal plane of scanning lens 805 with a magnification that gives the targeted spot size of the imaging beam 806 on the tissue. X-Y MEMS scanning mirror 803 deflects the beam so that the beam is scanned across the tissue for imaging, blood flow interruption or tissue cutting.

In certain examples of the present disclosure, a user interface may be implemented for a surgeon to operate the system. In particular examples, the user interface may be presented on a touchscreen display allowing the surgeon to examine OCT and/or MPL images of the tissue (*e.g.* a tumor as shown in FIGS. 11A and 11B, which represent en-face and B-scan cross-sectional images, respectively, of a skin cancer tumor) and specify a three-dimensional region for removal (*e.g.* dashed lines 171 and 172 as shown in FIGS. 11C and 11D). After the surgeon specifies a tissue region for removal, the procedure can start by interrupting (*e.g.* coagulating) blood flow in the most superficial 200 μm layer by raster scanning the 0.532 μm coagulation beam over the selected region. Directly after interrupting blood flow, the same 200 μm layer is removed by raster scanning the cutting beam over the same region (FIG. 11E). The process can be repeated so that blood flow in subsequent 200 μm tissue layer is first interrupted and then the tumor is removed. The surgical system may also be operated in a mode so that after the surgeon specifies a tissue region for removal, the procedure can start by interrupting (*e.g.* coagulating) blood flow in the most superficial 200 μm layer by raster scanning the 0.532 μm coagulation beam over the selected region. Directly after interrupting blood flow, the cutting beam can execute a circumcising cut with tissue inside the cut removed mechanically as practiced in the surgical art.

Operation and performance of examples may be validated in an animal model with a pathologic tissue. For example, using a subcutaneous xenograft tumor model in nude mice. HCT 116 cancer cells may be cultured in McCoy's 5A medium with 10% fetal bovine serum at 37°C under 5% $CO_2$. When culture reaches confluency, cells may be detached from the flask by 0.25% trypsin-EDTA, centrifuged, and re-suspended in sterile phosphate-buffered saline. Approximately $2 \times 10^6$ cells/50 μl may then be injected subcutaneously into the dorsal area of the mouse. After tumors grow to 1cm in diameter (approximately 2 weeks), a dorsal skin fold chamber can be mounted around the tumor region to position the animal for surgery.

Prior to surgery, dorsal skin containing the tumor may be raised from the mid-section of the body and sutured to an aluminum dorsal skin fold chamber to position the skin away from the body. A dorsal skin fold chamber model bearing tumor xenografts may be created in 30 female nude mice (NU/NU, Harlan, TX) divided into three groups. Group 1 animals (N=10) may serve as a negative control (no surgery). Group 2 animals (N=10) may serve as a positive control and undergo tumor resection with conventional surgery using either a scalpel, scissors or Bovie electrocautery. Group 3 animals (N=10) undergo surgery using examples according to the present disclosure. After two weeks, all animals in the three groups may be sacrificed with tumor regions cut for histology to compare treatment results. Efficacy of surgery using examples of the present disclosure vs. conventional surgery may be evaluated by examining histology for presence of any residual cancer and determining amount of damaged healthy tissue for mice in Groups 2 and 3, operating time, and ease to complete respective surgical procedures. Proceeding in this manner, operation and performance of examples can be validated against the conventional surgical procedure.

In another example, configuration for femoral neuroma removal may also be validated. Surgical removal of tumors inside the body cavity may be accomplished for example utilizing an endoscopic micro-applicator.

Optical design specifications of embodiments utilizing an endoscopic micro-applicator are similar to the bulk-applicator previously described. In particular, diameters of imaging ($D_{OCT}$ = 25 μm), coagulation ($D_G$ = 50 μm) and tissue removal ($D_C$ = 220 μm) beams on the tumor surface are equivalent to the bulk-applicator specifications.

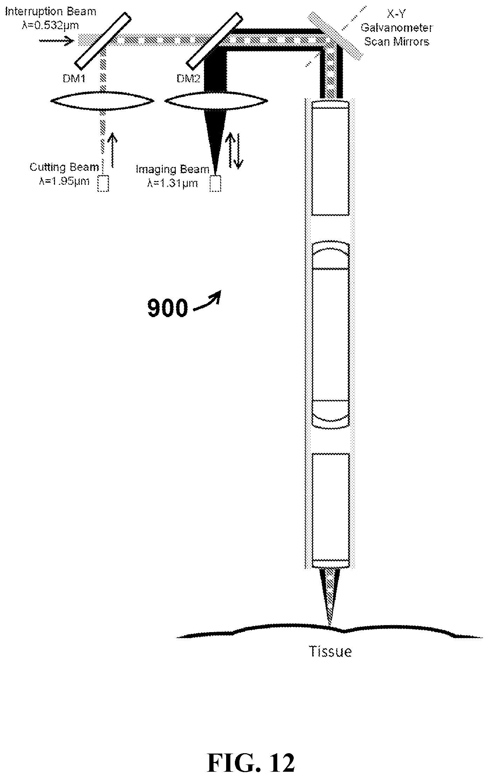

Certain examples may employ a modified Hopkins relay for design of the endoscopic micro-applicator. Using Zemax optical design software, a modified Hopkins relay design may be toleranced and optimized for imaging (1.32 μm), blood flow coagulation (0.532 nm) and tissue removal (1.95 μm) beams. Rod micro-lenses from glass blanks may be fabricated (*e.g.* by Optical Technology, Boston, MA). Before lens assembly, the rod micro-lenses may be anti-reflective coated (*e.g.* by Materion, Boston, MA). A high quality AR coating (*e.g.* < 0.2%, available from Materion) may be used for blood flow interruption, imaging and cutting beam wavelengths (0.532 μm, 1.32 μm, and 1.95 μm). After coating, the rod micro-lenses may be assembled in a 5 mm diameter stainless steel tube (*e.g.* with a 3 mm inside diameter). The stainless steel tube may be fastened to a stainless steel head manufactured by 3D rapid prototyping. The stainless steel head may house a scanning assembly similar to that utilized for the bulk applicator but including a telescope relay to scan collimated imaging, coagulation, and tissue removal beams at the front focal plane of the rod micro-lens assembly. Referring now to FIG. 12, an endoscopic micro-applicator 900 can be constructed by integrating the beam combiner and scanner with the endoscopic micro-lens assembly. A beam combiner assembly is interfaced to the endoscope or laproscope by imaging a conjugate plane of the scanning galvanometers to the front focal plane of the endoscope or laproscope using a focal system. Operation of the endoscopic or laproscopic micro-applicator may be verified using the protocol employed for the bulk applicator.

While one exemplary application includes removal of pathologic tissue in confined spaces surrounded by boney structures (*e.g.*, cholestetomas in the inner ear and inoperable brain tumors), an initial validation may utilize a simple model to minimize complications with animal preparation and survival after surgery. A VX2 rabbit cancer model was first proposed in 1933 and is used extensively. VX2 tumor fragments are used to establish a number of rabbit tumor models. The femoral nerve in the groin can be identified and easily distinguished from surrounding muscle and represents an attractive target site to induce a neuroma. This validation proposes to induce a femoral neuroma in a rabbit model to complete these studies.

Thirty-two adult New Zealand white male rabbits weighing 3.8 to 4.3 kg (Charles River, TN) are proposed to be enrolled in this study and divided into four groups. Group 1 (N = 2) rabbits serve as tumor donors. Group 2 (N = 10) and Group 3 (N=10) rabbits undergo percutaneous tumor implantation and tumor removal using either a conventional approach (Group 2) or our image-guided smart laser knife (Group 3). Group 4 (N = 10) rabbits serve as controls. To obtain tumor fragments for the implantation, rabbits in Group 1 will be injected with 125 mL of VX2 carcinoma cell suspension (NCI, MA) in each thigh muscle. Distinct solid tumors that grow in each thigh muscle will be harvested in two weeks, put into 4°C Hanks balanced salt solution, and then gently cut into 1 $mm^3$ fragments. The target site for tumor fragment implantation may then be selected in the groin region in muscle adjacent to the femoral nerve. To inject tumor fragments, animals in Groups 2 and 3 will undergo a conventional laparotomy through a 1cm incision in the groin region. An 18-gauge needle consisting of a cannula and core containing VX2 tumor fragments will be injected into muscle surrounding the femoral nerve and the incision sutured and animals allowed to recover. About one week after implantation when neuroma diameters are about 1 cm, rabbits in Group 2 will undergo conventional laparotomy with neuromas removed employing a conventional surgical approach using a scalpel, scissors or Bovie electrocautery. Rabbits in Group 3 will also undergo conventional laparotomy with neuromas removed using examples of the present disclosure through an endoscopic micro-applicator. After suturing the incisions, rabbits in Groups 2 and 3 will be observed and evaluated for impairment of locomotion and continence and compared with controls. After two weeks, all rabbits will be sacrificed and tissue regions containing the femoral nerve cut for histology to compare treatment results. Efficacy of examples to remove femoral neuromas through the endoscopic micro-applicator will be evaluated in relation to the conventional surgical procedure by examining for femoral nerve damage, presence of any residual cancer cells, and operating times to complete respective surgical procedures. Proceeding in this manner, operation and performance of examples to remove neuromas through an endoscopic micro-applicator can be validated against the conventional surgical procedure.

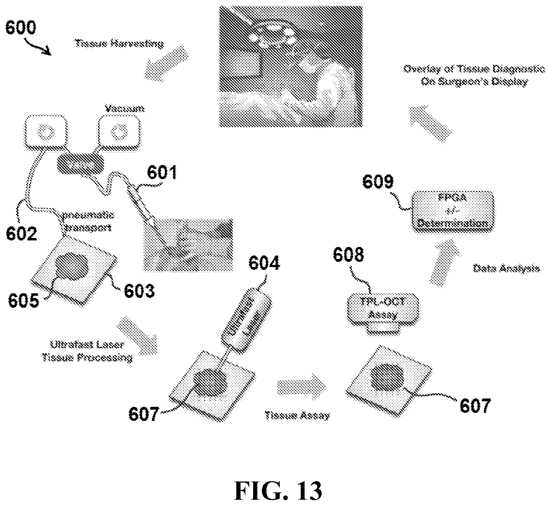

Examples of the present disclosure also comprise systems and methods for quickly establishing tumor margins with high sensitivity and specificity. Referring now to FIG. 13, a schematic of a laser-based optical biopsy system 600 is provided. An overview of the example shown in FIG. 13 provides the following procedural steps. Initially, on the surgeon's command (*e.g.* voice activation, foot pedal or push button) a small tissue specimen 605 (*e.g.* 1-4 $mm^3$) is harvested with a vacuum-assisted tool 601. The vacuum-assisted harvesting tool can be integrated with commonly-used surgical scalpels and/or cautery (*e.g.*, Bovie electrocautery) to streamline and maintain the surgeon's preferred workflow. Next, after vacuum-assisted harvest, the tissue specimen 605 can be transported through a positive-pressure fluidic channel 602 to a tissue processing chamber 603. Once in the tissue processing chamber 603, a laser 604 (*e.g.* an ultrafast femtosecond laser) can cut the tissue specimen 605 into a 500 $\mu$m thick slab section 607 for the image-based assay. A MPL-OCT optical assay 608 can then be performed on tissue slab 607. Data recorded by the MPL-OCT image-based assay 608 can then be processed by a field programmable gate array (FPGA) 609 that allows high speed diagnosis of the assayed tissue specimen. The result of the diagnostic analysis can then be communicated to the surgeon by projecting an overlay of the diagnosis on any of the various existing image modalities utilized by the surgeon. Thus, rather than only being able to process several frozen sections as is the current practice, the described optical chamber could process an order of magnitude greater samples, overlaying the results over a computer display of the entire operative field, providing the surgeon with knowledge of the presence of residual cancer along the entire surgical margin, currently not possible using traditional frozen sections.

System 600 can reduce the time needed to complete a diagnosis (*e.g.* less than three minutes) as compared to existing diagnostic systems. Accordingly, surgical operating times can be shortened, the surgeon's preferred work flow reinforced, and patient prognosis following surgery improved because the entire surgical margin can be mapped for residual cancer. Moreover, because the optical image-based diagnosis used in system 600 is nondestructive, traditional biopsy and histology can subsequently be performed on each harvested tissue slab to validate the diagnosis of system 600.

The MPL image-based assay used in system 600 requires properly-sized and shaped tissue specimens. Inasmuch as MPL and OCT image-based assays utilize rectilinear point-scanning approaches, optimal shape for tissue specimens compatible with these imaging modalities is a slab geometry. A processed tissue slab geometry is also compatible with conventional histological processing that may be completed following diagnosis in system 600.

Because MPL imaging depth is about 200-300 $\mu$m, imaging from both sides of the slab constrains the tissue specimen thickness for this approach to about 500 $\mu$m. Processing of the harvested tissue specimen should preserve tissue microstructure so that processing artifacts are not introduced into the MPL-OCT image-based assay.

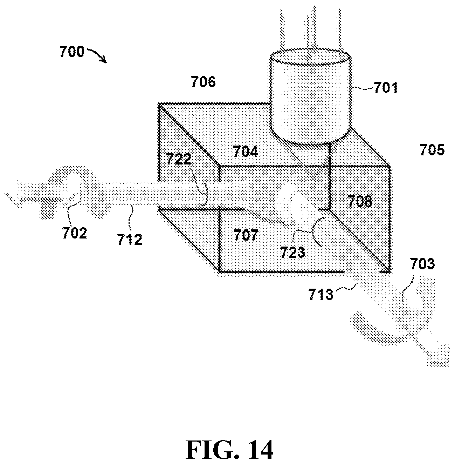

FIG. 14 shows a schematic of a tissue processing system 700 to process the harvested tissue into properly sized slabs for the MPL-OCT image-based assay. System 700 comprises an ultrafast fiber femtosecond laser 701 in combination with a vacuum-assisted tissue micro-position and hold actuators 702 and 703. Because the cutting action of the ultrafast laser 701 will utilize plasma ablation, properly sized and shaped tissue slabs 704 will have a negligible modified region at the slab surfaces and reduce the likelihood of the introduction of artifacts into the MPL-OCT imaged-based assay. A disposable tissue processing chamber 705 is proposed using vacuum assist tissue micro-position and hold actuators 702 and 703 for use in combination with scanning the ultrafast laser beam so that the harvested tissue can be rapidly processed into properly sized slabs for the image-based TPL-OCT assay.

As resected tissue transport will be done hydraulically, the tissue-processing chamber 705 can be filled with phosphate buffered saline 706. Transport of the tissue specimens 704 in and out of the tissue processing chamber 705 can occur via an inlet port 707 on one face and an outlet port 708 on the opposing side. A T-junction (not shown) upstream of the inlet and downstream of the outlet can be used to flush chamber 705 as needed to achieve the required optical transparency. Chamber 705 can have an optically clear window 709 for imaging and delivery of the beam from ultrafast laser 701. Chamber 705 can be disposable to prevent cross-contamination of the current patient's biopsy by previous patient biopsies. Tissue micro-position and hold actuators 702 and 703 can therefore engage and disengage from the chamber upon replacement. Two self-sealing rubber septa 722 and 723 can be incorporated into orthogonal facets of chamber 705 to form a water-tight seal for engagement/disengagement.

Actuators 702 and 703 can be housed in stainless steel needles 712 and 713 that will penetrate self-sealing rubber septa 722 and 723 built into tissue processing chamber 705. Two actuator / needle units can penetrate orthogonal sides of chamber 705 and can work together to move the tissue into any orientation. Once the tissue is in the chamber, a single actuator can penetrate the septum and a vacuum can be deployed. The actuator can be rotated and linearly translated via precision stepper motors to provide precise and accurate positioning of the specimen.

Vacuum suction can be applied to the hollow tube actuator at the same time saline is added to maintain a constant liquid volume. Once anchored to the actuator, the sample can be rotated fully about the deployed actuator axis and imaged using a visible wavelength camera. The second actuator can be deployed while the first actuator is disengaged, and the tissue can be imaged along the second actuator axis.

Optical systems interfacing with the tissue processing chamber can include an ultrafast laser, camera and OCT. An ultrafast laser (including for example, those available from Uranus-mJ, PolarOnyx Laser, USA) providing 1030 nm 500 fs pulses at a pulse energy of 0.5 mJ and repetition rate of 100 kHz on the specimen is utilized for rapid plasma ablation of tissue. The ultrafast laser beam can be combined with the OCT-MPL excitation beam by a dichroic mirror before the galvanometers. A CCD camera (including for example, acA1300-30gc, Basler, Germany) at the back aperture of the objective can be used for tissue specimen alignment in the lateral plane, while OCT can be employed for specimen alignment in the axial plane. The ultrafast laser beam can be scanned in combination with actuator movement to trim edges of the tissue specimen to create a planar slab geometry with 500 $\mu$m thickness.

Tissue processing system 700 can provide for efficient processing of tissue specimens and reduced processing times. A tissue specimen can be processed into a 500 $\mu$m thick planar slab geometry with two cut surfaces in less that sixty seconds (where the processing time is measured from the moment the tissue enters the processing chamber to the time when the ultrafast laser completes cutting the second planar interface).

The combined OCT-MPL fiber-based imaging systems (*e.g.* comprising swept-source OCT and MPL imaging modules and scanning optics) can be utilized to complete the image-based OCT-MPL assay.

An image-based OCT-MPL assay can be performed to demonstrate simultaneous detection of structure and composition of processed tissue slabs and diagnose whether cancer is present. Tumor tissues can be obtained from patients undergoing cancer surgery and MPL images recorded from both sides of the processed tissue slab and merged into a single volumetric image.

MPL images can indicate the presence of tissue constituents including lipids, calcium and collagen/elastin fibers. The energy state of the tissue can also be analyzed from the MPL signal including NADH and FAD+, which are involved in the Krebs cycle, since cancers will have an increased metabolic state compared to normal tissues. MPL emission spectra recorded (*e.g.* by photodetectors such as PMTs) can further distinguish these tissue types and redox states. Each co-registered *en face* OCT image can be merged with the MPL image to overlay biochemical composition onto the tissue OCT structural image. Inasmuch as OCT and MPL signals are due to two complementary types of optical contrast (*e.g.*, scattering and multiphoton absorption/emission, respectively), regions with strong/weak MPL emission correspond to weak/strong OCT signal, providing a comprehensive interpretation of the processed tissue block. A three-dimensional OCT dataset can also be merged with corresponding MPL images, demonstrating three-dimensional distributions of tissue constituents and energy states in relation to surface profile and structure.

After completion of the image-based MPL-OCT assay, tissue slabs can be processed using standard histology for the presence of cancer by a pathologist. By comparing the histological diagnosis with the image-based MPL-OCT assay a training set that identifies diagnostic parameters in MPL-OCT images can be determined using principal components analysis (PCA). A number of recent studies suggest that multiphoton luminescence (MPL) and optical coherence tomography (OCT) can be used to detect cancer.

## REFERENCES:

Heidt A, IEEE J. Select. Topics Quantum Electron, 20 (5), 2014.
El-Sherif A, Lasers Med Sci, 18, 139-147, 2003.
Richardson D, J. Opt. Soc. Am. B, 27, B63-B92, 2010.
Yusuf S, Circulation, 104, 2746-2753, 2001.
Libby P, Circulation, 105,1135-1143, 2002.
Libby P, Circulation, 111, 3481-3488, 2005.
Lucas AR, Circulation, 113, e728-732, 2006.
Virmani R, J Interv Cardiol, 16(3), 267-272, 2003.
Davies MJ, Br Heart J, 69, 377-381, 1993.
Stary HC, Circulation, 92, 1355-1374, 1995.
Jonasson L, Arteriosclerosis, 6, 131-138, 1986.
Johnson JL, Proc Natl Acad Sci, 102, 15575-15580, 2005.
Henney AM, Proc Natl Acad Sci, 88, 8154-8158, 1991.
Galis ZS, J Clin Invest, 94, 2493-2503, 1994.
Nikkari ST, Circulation, 92,1393-1398, 1995.
Libby P, Curr Opin Lipidol, 7, 330-335, 1996.
Taubman MB, **78**, 200-204, 1997.
Kolodgie FD, Heart, 90, 1385-1391, 2004.
van Zandvoort M, J Vasc Res, 41, 54-63, 2004.
Zoumi A, Biophys J, 87, 2778-2786, 2004.
Boulesteix T, Cytometry Part A, 69A, 20-26, 2006.

Le TT, J Biomed Opt, 12(5), 0540071-05400710, 2007.
Lilledahl MB, J Biomed Opt, 12(4), 0440051-04400512, 2007.
Wang T , J Biomed Opt, 17(3), 0360091-03600910, 2012.
Wang T, Lasers Surg Med, 44(1), 49-59, 2012.
Xue P, Chinese Science Bulletin, 53(13), 1963-1966, 2008.
Ryu SY, Opt Quant Electron, 37(13-15), 1191-1198, 2005.
Fu L, Opt Lett, 31, 1471-1473, 2006.
Liu G, J Biophoton, 4, 34-39, 2011.
Fu L, Opt Exp, 14, 1027-1032, 2006.
Wu Y, Opt Lett, 34, 953-955, 2009.
Kim, EH, Optics Communications, 249, 351-356, 2005.
Park J, Opt Exp, 16(3), 1590-1599, 2008.
V. L. Roger, Circulation, 123(4), e18-e209, 2011.
E. Falk, Circulation, 92(3), 657-671, 1995.
F. D. Kolodgie, Heart, 90(12), 1385-1391, 2004.
N. B. Hao, Clin. Dev. Immunol., 2012, 948098-948108, 2012.
B. Ruffell, Trends Immunol., 33(3), 119-126, 2012.
R. Shukla, Langmuir, 21(23), 10644-10654, 2005.
M. M. Janát-Amsbury, Eur. J. Pharm. Biopharm., 77(3), 417-423, 2011.
S. Lal, Acc. Chem. Res., 41(12), 1842-1851, 2008.
X. Ji, J. Phys. Chem. C, 111(17), 6245-6251, 2007.
S. E. Skrabalak, Nanomedicine (Lond), 2(5), 657-668, 2007.
M. Longmire, Nanomedicine (Lond), 3(5), 703-717, 2008.
L. L. Ma, ACS Nano, 3(9), 2686-2696, 2009.
T. Wang, Lasers Surg. Med., 44(1), 49-59, 2012.
T. S. Hauck, Small, 4(1), 153-159, 2008.
T. Niidome, J. Control Release, 114(3), 343-347, 2006.
A. Mooradian, Phys. Rev. Lett., 22(5), 185-187, 1969.
J. Zheng, Phys. Rev. Lett., 93(7), 077402-077405, 2004.
G. Wang, J. Am. Chem. Soc., 127(3), 812-813, 2005.
J. P. Wilcoxon, J. Chem. Phys., 108(21), 9137-9143, 1998.
Y. Fang, ACS Nano, 6(8), 7177-7184, 2012.
P. K. Jain, Plasmonics, 2(3), 107-118, 2007.
M. A. El-Sayed, Acc. Chem. Res., 34(4), 257-264, 2001.
C. Sönnichsen, Phys. Rev. Lett., 88, 077402-077405, 2002.
M. B. Mohamed, Chem. Phys. Lett., 317(6), 517-523, 2000.
S. Link, J. Phys. Chem., B 106(16), 3073-3077, 1999.
S. S. Verma, J. Optics, 41(2), 89-93, 2012.
P. K. Jain, Acc. Chem. Res., 41(12), 1578-1586, 2008.

E. T. Castellana, Langmuir, 26(8), 6066-6070, 2010.

H. Wang, Proc. Natl. Acad. Sci. USA ,102(44), 15752-15756, 2005.

L. Tong, Photochem. Photobiol., 85(1), 21-32, 2009.

T. Y. Ohulchanskyy, WIREs Nanomed. Nanobiotechnol., 2(2), 162-175, 2010.

D. Nagesha, Int. J. Nanomedicine, 2(4), 813-819, 2007.

Y. Zhang, J. Biomed. Opt., 15(2), 0205041-0205043, 2010.

C. L. Chen, Biomaterials, 31(14), 4104-4112, 2010.

H. Okamoto, J. Mater. Chem., 16(40), 3920-3928, 2006.

K. Imura, J. Phys. Chem. B , 109(27), 13214-13220, 2005.

W. H. Ni, ACS Nano, 2(4), 677-686, 2008.

C. Xu, JOSA B, 13(3), 481-491, 1996.

R. Gans, Ann. Phys., 47(10), 270-284, 1915.

M. A. Albota, Appl. Opt., 37(31), 7352-7356, 1998.

G.T. Boyd, Phys. Rev. B, 33(12), 7923-7936, 1986.

S. Eustis, J. Phys. Chem. B, 109(34), 16350-16356, 2005.

M. Guerrisi, Phys. Rev. B, 12(2), 557-563, 1975.

X. Huang, Adv. Mater., 21(48), 4880-4910, 2009.

K. S. Lee, J. Phys. Chem. B, 109(43), 20331-20338, 2005.

C. Sönnichsen, Nano Lett., 5(2), 301-304, 2005.

S. Link, J. Phys. Chem. B, 104(26), 6152-6163, 2000.

A. Bouhelier, Phys. Rev. Lett., 95(26), 2674051-2674054, 2005.

R. E. Hummel, Electronic Properties of Materials, 37-61, 2011.

R. Rosei, Phys. Rev. B, 12(2), 557-563, 1975.

Vakoc, Benjamin J., Nature medicine, 15.10, 1219-1223, 2009.

Perry, Seth W., Annals of biomedical engineering, 40.2, 277-291, 2012

**Claims**

1. A system comprising:

   a first light source (100) configured to provide a signal for use in imaging tissue when the first light source is incident upon tissue, wherein the first light source (100) comprises an optical coherence tomography light source;
   a second light source (200) configured to coagulate tissue when the second light source is incident upon tissue, wherein the second light source (200) is configured to emit energy in a range of wavelengths from 350 nm to 2200 nm; and
   a third light source (300) configured to break molecular bonds of tissue when the third light source is incident upon tissue; wherein

   the third light source (300) is a diode laser seeded fiber amplified source or a tunable semiconductor laser seeded fiber amplified source that is configured to emit energy in a range of wavelengths from 1800 nm to 2200 nm and in that the signal obtained from the first light source (100) is an input into a computer processor (360), and wherein the computer processor (360) provides output data used to control an orientation or position of the second light source (200) and the third light source (300), wherein the diode seeded fiber amplified source or the tunable semiconductor laser seeded fiber amplified source is configured to emit energy having a pulse profile, a pulse energy, and a pulse repetition rate, wherein at least one of the pulse profile, pulse energy and pulse repetition rate can be controlled to adjust a tissue removal rate.

2. The system of claim 1, wherein the third light source (300) is a Tm doped fiber master oscillator power amplifier (MOPA), preferably the Tm doped MOPA seed laser is a semiconductor diode laser or a tunable laser.

3. The system of claim 1, wherein the third light source (300) is configured to (a) break molecular bonds of tissue coagulated by the second light source (200) when the third light source (300) is incident upon tissue or (b) alter the quaternary structure of proteins of tissue when the third light source (300) is incident upon tissue.

4. The system of claim 1, wherein the first light source (100), the second light source (200) and the third light source (300) emit light through a single fiber (500) at the same instance or at different times, wherein single fiber preferably is a component of an endoscope or laproscope.

5. The system of claim 1, wherein the second light source (200) is a ytterbium fiber laser or a frequency-doubled ytterbium fiber laser.

6. The system of claim 1, wherein the second light source (200) is configured to emit energy in a range of wavelengths including 532 nm, 585 nm, 1064 nm and/or 1940nm.

7. The system of claim 1, wherein the first light source (100) comprises (a) a swept source optical coherence tomography light source or a broadband optical coherence tomography light source, and/or (b) a multiphoton luminescence light source.

8. The system of claim 1, wherein the second light source (200) is configured to emit energy at an amplitude and frequency sufficient to modify at least quaternary structure of tissue proteins without substantially breaking the molecular bonds of the tissue or wherein the third light source (300) is a laser configured to emit energy at an amplitude and frequency sufficient to break molecular bonds of tissue.

9. The system of claim 1, wherein the second light source (200) and the third light source (300) originate (a) from separate light sources or (b) from a common light source.

10. The system of claim 9, wherein the second light source (200) and the third light source (300) originate from a common light source at the same instance during use or from a common light source at different times during use.

11. A method of manipulating tissue ex vivo, the method comprising:

obtaining initial image signals of a first portion of tissue, wherein a first light source (100) is incident on the first portion of tissue; positioning a second light source (200) on a second portion tissue, wherein the second light source (200) coagulates the second portion of tissue; and breaking molecular bonds of a third portion of tissue via light energy, wherein the initial image signal obtained is used to position the second light source (200); wherein the steps of obtaining image signals, positioning the second light source (200) and breaking molecular bonds are performed using the system according to any one of claims 1 to 10.

**Patentansprüche**

1. Ein System bestehend aus: eine erste Lichtquelle (100), die so konfiguriert ist, dass sie ein Signal zur Verwendung bei der Abbildung von Gewebe liefert, wenn die erste Lichtquelle auf Gewebe einfällt, wobei die erste Lichtquelle (100) eine optische Kohärenztomographie-Lichtquelle umfasst; eine zweite Lichtquelle (200), die so konfiguriert ist, dass sie Gewebe koaguliert, wenn die zweite Lichtquelle auf Gewebe einfällt, wobei die zweite Lichtquelle (200) so konfiguriert ist, dass sie Energie in einem Wellenlängenbereich von 350 nm bis 2200 nm emittiert; und eine dritte Lichtquelle (300), die so konfiguriert ist, dass sie molekulare Bindungen von Gewebe aufbricht, wenn die dritte Lichtquelle auf Gewebe trifft; wobei die dritte Lichtquelle (300) eine mit einem Diodenlaser geimpfte faserverstärkte Quelle oder eine mit einem abstimmbaren Halbleiterlaser geimpfte faserverstärkte Quelle ist, die so konfiguriert ist, dass sie Energie in einem Wellenlängenbereich von 1800 nm bis 2200 nm emittiert, und dass das von der ersten Lichtquelle (100) erhaltene Signal eine Eingabe in einen Computerprozessor (360) ist, und wobei der Computerprozessor (360) Ausgabedaten bereitstellt, die zum Steuern einer Ausrichtung oder Position der zweiten Lichtquelle (200) und der dritten Lichtquelle (300) verwendet werden, wobei die mit einer Diode geimpfte Faserverstärkungsquelle oder die mit einem abstimmbaren Halbleiterlaser geimpfte Faserverstärkungsquelle so konfiguriert ist, dass sie Energie mit einem Pulsprofil, einer Pulsenergie, und einer Pulswiederholungsrate emittiert, wobei zumindest eines das Pulsprofil, die Pulsenergie und die Pulswiederholungsrate gesteuert werden kann, um die Gewebeentfernungsrate anzupassen.

2. System nach Anspruch 1, wobei die dritte Lichtquelle (300) ein Tm-dotierter Faser-Master-Oszillator-Leistungsverstärker (MOPA) ist, vorzugsweise ist der Tm-dotierte MOPA-Seed-Laser ein Halbleiterdiodenlaser oder ein abstimmbarer Laser.

3. System nach Anspruch 1, wobei die dritte Lichtquelle (300) so konfiguriert ist, dass sie (a) molekulare Bindungen von durch die zweite Lichtquelle (200) koaguliertem Gewebe aufbricht, wenn die dritte Lichtquelle (300) auf Gewebe trifft oder ( b) die Quartärstruktur von Gewebeproteinen verändern, wenn die dritte Lichtquelle (300) auf Gewebe trifft.

4. System nach Anspruch 1, wobei die erste Lichtquelle (100), die zweite Lichtquelle (200) und die dritte Lichtquelle (300) gleichzeitig oder zu unterschiedlichen Zeiten Licht durch eine einzelne Faser (500) emittieren, wobei die Einzelfaser vorzugsweise Bestandteil eines Endoskops oder Laproskops ist.

5. System nach Anspruch 1, wobei die zweite Lichtquelle (200) ein Ytterbium-Faserlaser oder ein frequenzverdoppelter Ytterbium-Faserlaser ist.

6. System nach Anspruch 1, wobei die zweite Lichtquelle (200) so konfiguriert ist, dass sie Energie in einem Wellenlängenbereich einschließlich 532 nm, 585 nm, 1064 nm und/oder 1940 nm emittiert.

7. System nach Anspruch 1, wobei die erste Lichtquelle (100) (a) eine Swept-Source-Lichtquelle für die optische Kohärenztomographie oder eine Breitband-Lichtquelle für die optische Kohärenztomographie und/oder (b) eine Multiphotonen-Lumineszenzlichtquelle umfasst.

8. System nach Anspruch 1, wobei die zweite Lichtquelle (200) so konfiguriert ist, dass sie Energie mit einer Amplitude und Frequenz emittiert, die ausreicht, um zumindest die Quartärstruktur von Gewebeproteinen zu modifizieren, ohne die molekularen Bindungen des Gewebes wesentlich aufzubrechen, oder wobei die dritte Lichtquelle (300) ein Laser ist, der so konfiguriert ist, dass er Energie mit einer Amplitude und Frequenz abgibt, die ausreicht, um molekulare Bindungen im Gewebe aufzubrechen.

9.  System nach Anspruch 1, wobei die zweite Lichtquelle (200) und die dritte Lichtquelle (300) (a) von separaten Lichtquellen oder (b) von einer gemeinsamen Lichtquelle stammen.

10. System nach Anspruch 9, wobei die zweite Lichtquelle (200) und die dritte Lichtquelle (300) zum gleichen Zeitpunkt während der Verwendung von einer gemeinsamen Lichtquelle oder zu unterschiedlichen Zeiten während der Verwendung von einer gemeinsamen Lichtquelle stammen.

11. Verfahren zur Manipulation von Gewebe ex vivo, wobei das Verfahren folgendes umfasst: Erhalten erster Bildsignale eines ersten Gewebeabschnitts, wobei eine erste Lichtquelle (100) auf den ersten Gewebeabschnitt fällt; Positionieren einer zweiten Lichtquelle (200) auf einem zweiten Gewebeabschnitt, wobei die zweite Lichtquelle (200) den zweiten Gewebeabschnitt koaguliert; und Aufbrechen molekularer Bindungen eines dritten Gewebeabschnitts mittels Lichtenergie, wobei das erhaltene anfängliche Bildsignal zum Positionieren der zweiten Lichtquelle (200) verwendet wird; wobei die Schritte des Erhaltens von Bildsignalen, des Positionierens der zweiten Lichtquelle (200) und des Aufbrechens von molekularen Bindungen unter Verwendung des Systems nach einem der Ansprüche 1 bis 10 durchgeführt werden.

**Revendications**

1.  Système comprenant :

    une première source de lumière (100) conçue pour fournir un signal pour l'utilisation dans l'imagerie d'un tissu lorsque la première source de lumière est incidente sur un tissu, la première source de lumière (100) comprenant une source de lumière de tomographie par cohérence optique ;
    une seconde source de lumière (200) conçue pour coaguler un tissu lorsque la seconde source de lumière est incidente sur un tissu, la seconde source de lumière (200) étant conçue pour émettre de l'énergie dans une plage de longueurs d'ondes de 350 nm à 2 200 nm ; et
    une troisième source de lumière (300) conçue pour briser des liaisons moléculaires de tissu lorsque la troisième source de lumière est incidente sur un tissu ;

la troisième source de lumière (300) étant une source amplifiée par fibre d'injection à diode laser ou une source amplifiée par fibre d'injection laser à semi-conducteur accordable qui est conçue pour émettre de l'énergie dans une plage de longueurs d'ondes de 1 800 nm à 2 200 nm et en ce que le signal obtenu à partir de la première source de lumière (100) est une saisie dans un processeur informatique (360), et le processeur informatique (360) fournissant des données de sortie utilisées pour commander une orientation ou une position de la seconde source de lumière (200) et de la troisième source de lumière (300), la source amplifiée par fibre d'injection à diode ou la source amplifiée par fibre d'injection laser à semi-conducteur accordable étant conçue pour émettre de l'énergie ayant un profil d'impulsion, une énergie d'impulsion, et un taux de répétition d'impulsion, au moins l'un du profil d'impulsion, de l'énergie d'impulsion et du taux de répétition d'impulsion pouvant être régulé pour ajuster un taux d'élimination d'un tissu.

2.  Système selon la revendication 1, la troisième source de lumière (300) étant un amplificateur de puissance à oscillateur maître (MOPA), préférablement le laser à injection MOPA dopé au Tm étant un laser à diode semi-conductrice ou un laser accordable.

3.  Système selon la revendication 1, la troisième source de lumière (300) étant conçue pour (a) briser des liaisons moléculaires de tissu coagulé par la seconde source de lumière (200) lorsque la troisième source de lumière (300) est incidente sur un tissu ou (b) altérer la structure quaternaire des protéines de tissu lorsque la troisième source de lumière (300) est incidente sur un tissu.

4.  Système selon la revendication 1, la première source de lumière (100), la seconde source de lumière (200) et la troisième source de lumière (300) émettant de la lumière à travers une fibre unique (500) en même temps ou à des moments différents, une fibre unique étant préférablement un composant d'un endoscope ou d'un laproscope.

5.  Système selon la revendication 1, la seconde source de lumière (200) étant un laser à fibre d'ytterbium ou un laser à fibre d'ytterbium à fréquence doublée.

6.  Système selon la revendication 1, la seconde source de lumière (200) étant conçue pour émettre de l'énergie dans une plage de longueurs d'ondes incluant 532 nm, 585 nm, 1 064 nm et/ou 1 940 nm.

7.  Système selon la revendication 1, la première source de lumière (100) comprenant (a) une source de lumière de tomographie par cohérence optique à source de signaux balayée ou une source de lumière de tomographie par cohérence optique à large bande, et/ou (b) une source de lumière de luminescence multiphoton.

8. Système selon la revendication 1, la seconde source de lumière (200) étant conçue pour émettre de l'énergie à une amplitude et une fréquence suffisantes pour modifier au moins une structure quaternaire de protéines tissulaires sans briser sensiblement les liaisons moléculaires du tissu ou la troisième source de lumière (300) étant un laser conçu pour émettre de l'énergie à une amplitude et à une fréquence suffisantes pour briser des liaisons moléculaires de tissu.

9. Système selon la revendication 1, la seconde source de lumière (200) et la troisième source de lumière (300) provenant (a) de sources de lumière séparées ou (b) d'une source de lumière commune.

10. Système selon la revendication 9, la seconde source de lumière (200) et la troisième source de lumière (300) provenant d'une source de lumière commune au même moment durant l'utilisation ou depuis une source de lumière commune à différents moments durant une utilisation.

11. Procédé de manipulation de tissu ex *vivo,* le procédé consistant à :

   obtenir des signaux d'image initiaux d'une première portion de tissu, une première source de lumière (100) étant incidente sur la première portion de tissu ;
   positionner une seconde source de lumière (200) sur une seconde portion de tissu, la seconde source de lumière (200) coagulant la seconde portion de tissu ; et
   à briser des liaisons moléculaires d'une troisième portion de tissu par l'intermédiaire d'une énergie lumineuse, le signal d'image initial obtenu étant utilisé pour positionner la seconde source de lumière (200) ;
   les étapes d'obtention des signaux d'image, de positionnement de la seconde source de lumière (200) et de rupture des liaisons moléculaires étant effectuées en utilisant le système selon l'une quelconque des revendications 1 à 10.

**FIG. 1**

**FIG. 2**

EP 3 191 005 B1

FIG. 3

22

**FIG. 4**

**FIG. 5A**

**FIG. 5B**

**FIG. 5C**

**FIG. 6**

**FIG. 7**

FIG. 8

**FIG. 9**

**FIG. 10**

**FIG. 11**

Interruption Beam
λ=0.532μm

DM1

DM2

X-Y
Galvanometer
Scan Mirrors

Cutting Beam
λ=1.95μm

Imaging Beam
λ=1.31μm

$900$

Tissue

**FIG. 12**

**FIG. 13**

FIG. 14

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 5451221 A
- US 2011257641 A1
- US 7415049 B

## Non-patent literature cited in the description

- **ALEXANDER M. HEIDT ; ZHIHONG LI ; DAVID J. RICHARDSON.** High Power Diode-Seeded Fiber Amplifiers at 2 μm-From Architectures to Applications. *IEEE J. Select. Topics Quantum Electron.,* September 2014, vol. 20 (5
- **HEIDT A.** *IEEE J. Select. Topics Quantum Electron,* 2014, vol. 20 (5
- **EL-SHERIF A.** *Lasers Med Sci,* 2003, vol. 18, 139-147
- **RICHARDSON D.** *J. Opt. Soc. Am. B,* 2010, vol. 27, B63-B92
- **YUSUF S.** *Circulation,* 2001, vol. 104, 2746-2753
- **LIBBY P.** *Circulation,* 2002, vol. 105, 1135-1143
- **LIBBY P.** *Circulation,* 2005, vol. 111, 3481-3488
- **LUCAS AR.** *Circulation,* 2006, vol. 113, e728-732
- **VIRMANI R.** *J Interv Cardiol,* 2003, vol. 16 (3), 267-272
- **DAVIES MJ.** *Br Heart J,* 1993, vol. 69, 377-381
- **STARY HC.** *Circulation,* 1995, vol. 92, 1355-1374
- **JONASSON L.** *Arteriosclerosis,* 1986, vol. 6, 131-138
- **JOHNSON JL.** *Proc Natl Acad Sci,* 2005, vol. 102, 15575-15580
- **HENNEY AM.** *Proc Natl Acad Sci,* 1991, vol. 88, 8154-8158
- **GALIS ZS.** *J Clin Invest,* 1994, vol. 94, 2493-2503
- **NIKKARI ST.** *Circulation,* 1995, vol. 92, 1393-1398
- **LIBBY P.** *Curr Opin Lipidol,* 1996, vol. 7, 330-335
- **KOLODGIE FD.** *Heart,* 2004, vol. 90, 1385-1391
- **VAN ZANDVOORT M.** *J Vasc Res,* 2004, vol. 41, 54-63
- **ZOUMI A.** *Biophys J,* 2004, vol. 87, 2778-2786
- **BOULESTEIX T.** *Cytometry Part A,* 2006, vol. 69A, 20-26
- **LE TT.** *J Biomed Opt,* 2007, vol. 12 (5), 0540071-05400710
- **LILLEDAHL MB.** *J Biomed Opt,* 2007, vol. 12 (4), 0440051-04400512
- **WANG T.** *J Biomed Opt,* 2012, vol. 17 (3), 0360091-03600910
- **WANG T.** *Lasers Surg Med,* 2012, vol. 44 (1), 49-59
- **XUE P.** *Chinese Science Bulletin,* 2008, vol. 53 (13), 1963-1966
- **RYU SY.** *Opt Quant Electron,* 2005, vol. 37 (13-15), 1191-1198
- **FU L.** *Opt Lett,* 2006, vol. 31, 1471-1473
- **LIU G.** *J Biophoton,* 2011, vol. 4, 34-39
- **FU L.** *Opt Exp,* 2006, vol. 14, 1027-1032
- **WU Y.** *Opt Lett,* 2009, vol. 34, 953-955
- **KIM, EH.** *Optics Communications,* 2005, vol. 249, 351-356
- **PARK J.** *Opt Exp,* 2008, vol. 16 (3), 1590-1599
- **V. L. ROGER.** *Circulation,* 2011, vol. 123 (4), e18-e209
- **E. FALK.** *Circulation,* 1995, vol. 92 (3), 657-671
- **F. D. KOLODGIE.** *Heart,* 2004, vol. 90 (12), 1385-1391
- **N. B. HAO.** *Clin. Dev. Immunol.,* 2012, vol. 2012, 948098-948108
- **B. RUFFELL.** *Trends Immunol.,* 2012, vol. 33 (3), 119-126
- **R. SHUKLA.** *Langmuir,* 2005, vol. 21 (23), 10644-10654
- **M. M. JANÁT-AMSBURY.** *Eur. J. Pharm. Biopharm.,* 2011, vol. 77 (3), 417-423
- **S. LAL.** *Acc. Chem. Res.,* 2008, vol. 41 (12), 1842-1851
- **X. JI.** *J. Phys. Chem. C,* 2007, vol. 111 (17), 6245-6251
- **S. E. SKRABALAK.** *Nanomedicine (Lond),* 2007, vol. 2 (5), 657-668

- **M. LONGMIRE.** *Nanomedicine (Lond),* 2008, vol. 3 (5), 703-717
- **L. L. MA.** *ACS Nano,* 2009, vol. 3 (9), 2686-2696
- **T. WANG.** *Lasers Surg. Med.,* 2012, vol. 44 (1), 49-59
- **T. S. HAUCK.** *Small,* 2008, vol. 4 (1), 153-159
- **T. NIIDOME.** *J. Control Release,* 2006, vol. 114 (3), 343-347
- **A. MOORADIAN.** *Phys. Rev. Lett.,* 1969, vol. 22 (5), 185-187
- **J. ZHENG.** *Phys. Rev. Lett.,* 2004, vol. 93 (7), 077402-077405
- **G. WANG.** *J. Am. Chem. Soc,* 2005, vol. 127 (3), 812-813
- **J. P. WILCOXON.** *J. Chem. Phys.,* 1998, vol. 108 (21), 9137-9143
- **Y. FANG.** *ACS Nano,* 2012, vol. 6 (8), 7177-7184
- **P. K. JAIN.** *Plasmonics,* 2007, vol. 2 (3), 107-118
- **M. A. EL-SAYED.** *Acc. Chem. Res.,* 2001, vol. 34 (4), 257-264
- **C. SÖNNICHSEN.** *Phys. Rev. Lett.,* 2002, vol. 88, 077402-077405
- **M. B. MOHAMED.** *Chem. Phys. Lett,* 2000, vol. 317 (6), 517-523
- **S. LINK.** *J. Phys. Chem., B,* 1999, vol. 106 (16), 3073-3077
- **S. S. VERMA.** *J. Optics,* 2012, vol. 41 (2), 89-93
- **P. K. JAIN.** *Acc. Chem. Res.,* 2008, vol. 41 (12), 1578-1586
- **E. T. CASTELLANA.** *Langmuir,* 2010, vol. 26 (8), 6066-6070
- **H. WANG.** *Proc. Natl. Acad. Sci. USA,* 2005, vol. 102 (44), 15752-15756
- **L. TONG.** *Photochem. Photobiol.,* 2009, vol. 85 (1), 21-32
- **T. Y. OHULCHANSKYY.** *WIREs Nanomed. Nanobiotechnol.,* 2010, vol. 2 (2), 162-175
- **D. NAGESHA.** *Int. J. Nanomedicine,* 2007, vol. 2 (4), 813-819
- **Y. ZHANG.** *J. Biomed. Opt.,* 2010, vol. 15 (2), 0205041-0205043
- **C. L. CHEN.** *Biomaterials,* 2010, vol. 31 (14), 4104-4112
- **H. OKAMOTO.** *J. Mater. Chem.,* 2006, vol. 16 (40), 3920-3928
- **K. IMURA.** *J. Phys. Chem. B,* 2005, vol. 109 (27), 13214-13220
- **W. H. NI.** *ACS Nano,* 2008, vol. 2 (4), 677-686
- **C. XU.** *JOSA B,* 1996, vol. 13 (3), 481-491
- **R. GANS.** *Ann. Phys.,* 1915, vol. 47 (10), 270-284
- **M. A. ALBOTA.** *Appl. Opt.,* 1998, vol. 37 (31), 7352-7356
- **G.T. BOYD.** *Phys. Rev. B,* 1986, vol. 33 (12), 7923-7936
- **S. EUSTIS.** *J. Phys. Chem. B,* 2005, vol. 109 (34), 16350-16356
- **M. GUERRISI.** *Phys. Rev. B,* 1975, vol. 12 (2), 557-563
- **X. HUANG.** *Adv. Mater.,* 2009, vol. 21 (48), 4880-4910
- **K. S. LEE.** *J. Phys. Chem. B,* 2005, vol. 109 (43), 20331-20338
- **C. SÖNNICHSEN.** *Nano Lett.,* 2005, vol. 5 (2), 301-304
- **S. LINK.** *J. Phys. Chem. B,* 2000, vol. 104 (26), 6152-6163
- **A. BOUHELIER.** *Phys. Rev. Lett.,* 2005, vol. 95 (26), 2674051-2674054
- **R. E. HUMMEL.** *Electronic Properties of Materials,* 2011, 37-61
- **R. ROSEI.** *Phys. Rev. B,* 1975, vol. 12 (2), 557-563
- **VAKOC, BENJAMIN J.** *Nature medicine,* 2009, vol. 15 (10), 1219-1223
- **PERRY, SETH W.** *Annals of biomedical engineering,* 2012, vol. 40 (2), 277-291